(19) Europäisches Patentamt
European Patent Office
Office européen des brevets

(11) EP 4 796 536 A1

(12) **EUROPEAN PATENT APPLICATION**
published in accordance with Art. 153(4) EPC

(43) Date of publication:
**26.08.2026 Bulletin 2026/35**

(21) Application number: **24902905.9**

(22) Date of filing: **12.12.2024**

(51) International Patent Classification (IPC):
***C07D 401/14** (2006.01)* ***A61K 31/519** (2006.01)*
***A61P 37/06** (2006.01)* ***A61P 29/00** (2006.01)*
***A61P 35/00** (2006.01)* ***A61P 9/00** (2006.01)*
***A61P 25/00** (2006.01)* ***A61P 17/06** (2006.01)*
***A61P 19/02** (2006.01)* ***A61P 9/10** (2006.01)*

(52) Cooperative Patent Classification (CPC):
**A61K 31/519; A61P 9/00; A61P 9/10; A61P 17/06; A61P 19/02; A61P 25/00; A61P 29/00; A61P 35/00; A61P 37/06; C07D 401/14**

(86) International application number:
**PCT/CN2024/138851**

(87) International publication number:
**WO 2025/124488 (19.06.2025 Gazette 2025/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.12.2023 CN 202311712765**
**18.11.2024 CN 202411656766**

(71) Applicant: **Hangzhou Polymed Biopharmaceuticals, Inc.**
**Hangzhou, Zhejiang 311100 (CN)**

(72) Inventors:
- **XIANG, Jason Shaoyun**
**Hangzhou, Zhejiang 311100 (CN)**
- **WU, Lei**
**Hangzhou, Zhejiang 311100 (CN)**

(74) Representative: **Canzler & Bergmeier Patentanwälte Partnerschaft mbB**
**Despag-Straße 6**
**85055 Ingolstadt (DE)**

# (54) CRYSTAL FORMS OF COMPOUND HAVING FUSED FIVE- AND SIX-MEMBERED RINGS, PREPARATION METHOD THEREFOR, AND USE THEREOF

(57) Provided are crystal forms of a compound having fused five- and six-membered rings, a preparation method therefor, and the use thereof. Particularly provided are a crystal form A, a crystal form B, a crystal form C, a crystal form D, a crystal form F, a crystal form G, a crystal form H, a crystal form I or a crystal form J of said compound represented by formula X. The X-ray powder diffraction pattern of the crystal form A using Cu-Kα radiation and represented by 2θ has diffraction peaks at 6.9±0.2°, 11.2±0.2°, 12.2±0.2°, 13.7±0.2°, 17.3 ±0.2°, 18.2±0.2° and 24.3±0.2°. The crystal forms of said compound have IRAK4 inhibition or/and degradation effects, have potential value in clinical application, and are expected to be capable of improving the prognosis of patients and reducing the possibility of drug resistance; in addition, said crystal forms have good solubility, physical and chemical stability and mechanical stability.

Intensity
2θ(°)

FIG. 2

EP 4 796 536 A1

## Description

The present application claims the right of the priorities of Chinese patent application 2023117127653 filed on December 13, 2023 and Chinese patent application 202411656766.5 filed on November 18, 2024. The contents of the above Chinese patent applications are incorporated herein by reference in their entirety.

TECHNICAL FIELD

The present disclosure relates to a crystal form of a five-membered-fused six-membered compound, a preparation method therefor, and a use thereof.

BACKGROUND

Kinases have always been important therapeutic targets for the development of antiinflammatory drugs (Current Opinion in Cell Biology 2009 21, 1-8). Interleukin-1 receptor-associated kinases (IRAKs) are serine/threonine protein kinases that belong to the tyrosine-like kinase (TLK) family. IRAKs are located downstream of toll-like receptor and IL-1R pathways, among which IRAK1 and IRAK4 have kinase activity. IRAK4 acts upstream of the IRAK family kinase activation pathway and plays an important role in innate immune signaling (Science 1996, 271(5252): 1128-31). Stimulation of TLR can recruit myeloid differentiation primary response 88 (MYD88) and activate receptors to form a complex, Myddosome, which then forms a complex with IRAK4 to activate IRAK1. Subsequently, TRAF6 is activated by IRAK1, leading to the activation of the NF-κB and AMPK signaling pathways, which ultimately results in the expression of inflammatory cytokines (Molecules 2016, 21, 1529, J Biol Chem. 2018 Sep 28; 293(39): 15195-15207, Eur J. Immunol. 2008. 38: 614-618).

As a very important feature, IRAK4 has two functions of scaffolding and kinase phosphorylation in the TLR and IL-1R signaling pathways. The kinase domain (KD) provides the kinase function, and the death domain (DD) provides the scaffolding function for Myddosome (Molecules 2016, 21(11), 1529). Myddosome is related to a variety of diseases, not only to autoimmune and inflammatory diseases but also to cancer. For example, MYD88 mutations account for 39% of patients with active B-cell-like diffuse large B-cell lymphoma (ABC DLBCL) and 86% to 100% of patients with several other types of B-cell malignancies and primary central nervous system lymphomas (Cell Chemical Biology 27, 1-10, December 17, 2020).

IRAK4 knockout mice and clinicopathologic studies have shown that IRAK4 deficiency itself is nonlethal, and that individuals with IRAK4 mutations are also been protected from chronic lung disease and inflammatory bowel disease (Eur. J. Immunol. 2008. 38: 614-618). IRAK4 inhibitors have been considered as targets for the treatment of immune diseases, such as autoimmune diseases, including rheumatoid arthritis (RA), systemic lupus erythematosus (SLE), and psoriasis (Expert Opinion on Therapeutic Patents Volume 29, 2019-Issue 4). Meanwhile, IRAK4 is also a popular target for the treatment of tumors, and a few IRAK4 kinase inhibitors have entered the clinical stage. However, these drugs under development in clinical stages are all inhibitors with the kinase function (KD) of IRAK4 and have no direct inhibitory effect on the scaffolding function of IRAK4. Proteolysis-targeting chimeras (PROTACs) targeting IRAK4 are expected to simultaneously eliminate its kinase activity and scaffolding function, resulting in better and broader efficacy (Nature Biotechnology 2020, volume 38, pages 1221-1223, ACS Med Chem Lett. 2019 Jul 11; 10(7): 1081-1085).

CONTENT OF THE PRESENT INVENTION

The present disclosure discloses a crystal form of a five-membered-fused six-membered compound, a preparation method therefor, and a use thereof. The crystal form of the five-membered-fused six-membered compound of the present disclosure has an inhibitory or/and degrading effect on IRAK4 with potential clinical application value, and is expected to improve the prognosis of patients and reduce the possibility of drug resistance. Moreover, it also has good solubility, physicochemical stability, and mechanical stability.

The present disclosure solves the above technical problems by the following technical solutions.

The present disclosure provides a crystal form A of a five-membered-fused six-membered compound of formula X, which has an X-ray powder diffraction (XRPD) pattern using Cu-Kα radiation and represented by 2θ angles comprising diffraction peaks at 6.9 ± 0.2°, 11.2 ± 0.2°, 12.2 ± 0.2°, 13.7 ± 0.2°, 17.3 ± 0.2°, 18.2 ± 0.2°, and 24.3 ± 0.2°;

X

In a certain embodiment, the X-ray powder diffraction (XRPD) pattern for the crystal form A, using Cu-Kα radiation and represented by 2θ angles, further comprises diffraction peaks at one or more of 9.7 ± 0.2°, 10.2 ± 0.2°, 15.1 ± 0.2°, and 20.6 ± 0.2°.

In a certain embodiment, the X-ray powder diffraction (XRPD) pattern for the crystal form A, using Cu-Kα radiation and represented by 2θ angles, has positions of diffraction peaks as shown in the following table:

| No. | Angle (°) | No. | Angle (°) |
|---|---|---|---|
| 1 | 6.9 | 9 | 17.3 |
| 2 | 9.7 | 10 | 18.2 |
| 3 | 10.2 | 11 | 18.3 |
| 4 | 11.2 | 12 | 20.6 |
| 5 | 12.2 | 13 | 21.6 |
| 6 | 13.7 | 14 | 24.3 |
| 7 | 15.1 | 15 | 24.8 |
| 8 | 16.4 | | |

In a certain embodiment, the X-ray powder diffraction (XRPD) pattern for the crystal form A, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.9 | 63.6% | 9 | 17.3 | 43.2% |
| 2 | 9.7 | 18.3% | 10 | 18.2 | 52.4% |
| 3 | 10.2 | 16.2% | 11 | 18.3 | 47.0% |
| 4 | 11.2 | 60.1% | 12 | 20.6 | 32.7% |
| 5 | 12.2 | 29.2% | 13 | 21.6 | 29.8% |
| 6 | 13.7 | 100.0% | 14 | 24.3 | 27.6% |
| 7 | 15.1 | 23.2% | 15 | 24.8 | 19.8% |
| 8 | 16.4 | 20.8% | | | |

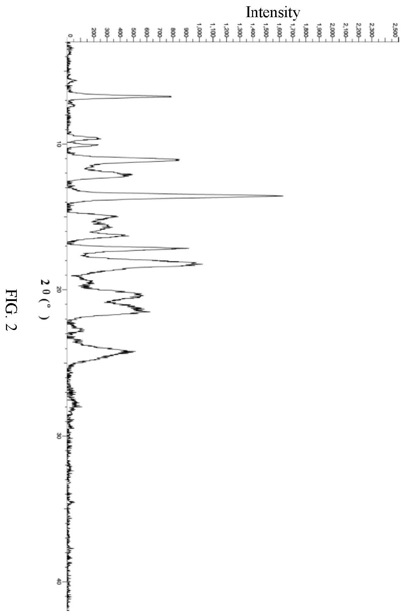

In a certain embodiment, the X-ray powder diffraction (XRPD) pattern for the crystal form A, using Cu-Kα radiation and represented by 2θ angles, is substantially as shown in FIG. 2.

In a certain embodiment, the crystal form A has a differential scanning calorimetry (DSC) pattern comprising an endothermic peak at 262.80 ± 5°C.

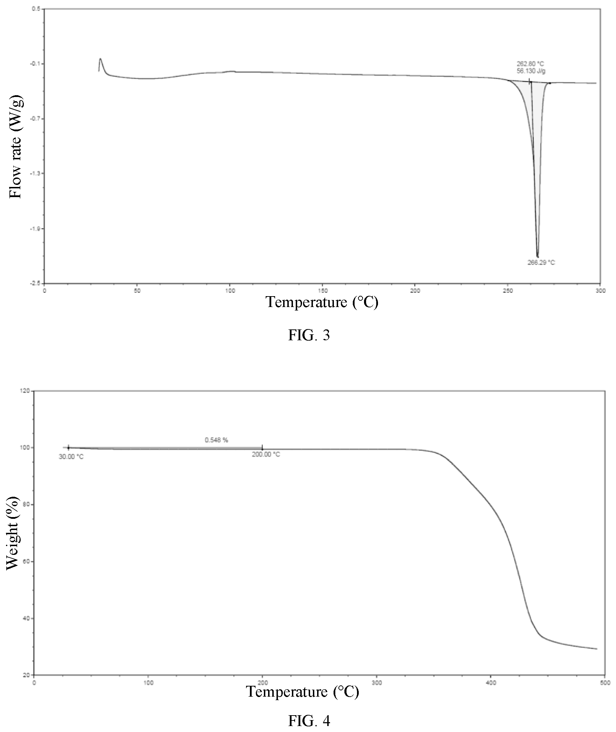

In a certain embodiment, the differential scanning calorimetry (DSC) pattern for the crystal form A is substantially as shown in FIG. 3.

In a certain embodiment, the crystal form A has a thermogravimetric analysis (TGA) pattern with a weight loss of 0.548% before 200 ± 5°C.

In a certain embodiment, the thermogravimetric analysis (TGA) pattern for the crystal form A is substantially as

shown in FIG. 4.

The present disclosure further provides a crystal form C of the five-membered-fused six-membered compound of formula X, which has an X-ray powder diffraction (XRPD) pattern using Cu-Kα radiation and represented by 2θ angles comprising diffraction peaks at 9.7 ± 0.2°, 10.8 ± 0.2°, 13.1 ± 0.2°, 14.1 ± 0.2°, 18.7 ± 0.2°, and 21.7 ± 0.2°;

X

.

In a certain embodiment, the X-ray powder diffraction (XRPD) pattern for the crystal form C, using Cu-Kα radiation and represented by 2θ angles, further comprises diffraction peaks at one or more of 12.5 ± 0.2°, 15.5 ± 0.2°, and 24.3 ± 0.2°.

In a certain embodiment, the X-ray powder diffraction (XRPD) pattern for the crystal form C, using Cu-Kα radiation and represented by 2θ angles, has positions of diffraction peaks as shown in the following table:

| No. | Angle (°) | No. | Angle (°) |
|---|---|---|---|
| 1 | 9.7 | 9 | 16.8 |
| 2 | 10.8 | 10 | 18.7 |
| 3 | 12.5 | 11 | 19.6 |
| 4 | 13.1 | 12 | 19.8 |
| 5 | 14.1 | 13 | 20.7 |
| 6 | 14.7 | 14 | 21.7 |
| 7 | 15.2 | 15 | 22.4 |
| 8 | 15.5 | 16 | 24.3 |

In a certain embodiment, the X-ray powder diffraction (XRPD) pattern for the crystal form C, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 9.7 | 34.5% | 9 | 16.8 | 21.6% |
| 2 | 10.8 | 25.5% | 10 | 18.7 | 100.0% |
| 3 | 12.5 | 29.3% | 11 | 19.6 | 55.4% |
| 4 | 13.1 | 35.7% | 12 | 19.8 | 57.1% |
| 5 | 14.1 | 48.6% | 13 | 20.7 | 69.5% |
| 6 | 14.7 | 12.3% | 14 | 21.7 | 27.0% |
| 7 | 15.2 | 22.6% | 15 | 22.4 | 45.7% |
| 8 | 15.5 | 33.4% | 16 | 24.3 | 13.1% |

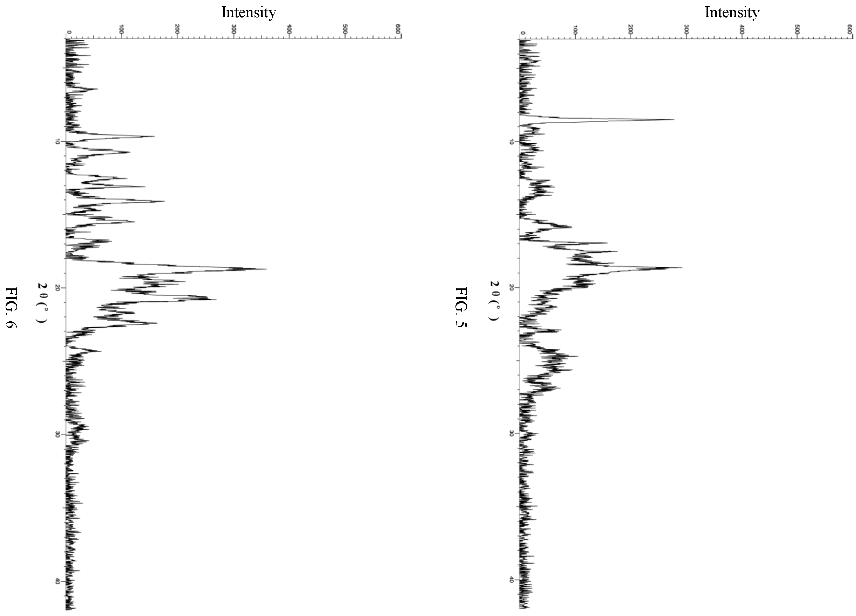

In a certain embodiment, the X-ray powder diffraction (XRPD) pattern for the crystal form C, using Cu-Kα radiation and represented by 2θ angles, is substantially as shown in FIG. 6.

In a certain embodiment, the crystal form C has a differential scanning calorimetry (DSC) pattern comprising an

endothermic peak at 261.8 ± 5°C and an exothermic peak at 266.8 ± 5°C.

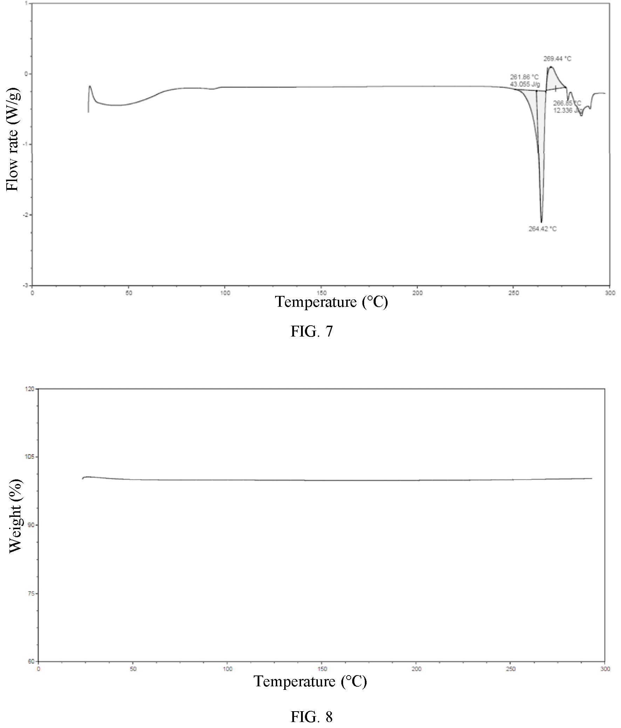

In a certain embodiment, the differential scanning calorimetry (DSC) pattern for the crystal form C is substantially as shown in FIG. 7.

In a certain embodiment, the crystal form C has a thermogravimetric analysis (TGA) pattern with essentially no weight loss before 300 ± 5°C.

In a certain embodiment, the thermogravimetric analysis (TGA) pattern for the crystal form C is substantially as shown in FIG. 8.

The present disclosure further provides a crystal form F of the five-membered-fused six-membered compound of formula X, which has an X-ray powder diffraction (XRPD) pattern using Cu-Kα radiation and represented by 2θ angles comprising diffraction peaks at 5.8 ± 0.2°, 6.6 ± 0.2°, 7.2 ± 0.2°, 12.2 ± 0.2°, 14.5 ± 0.2°, 15.7 ± 0.2°, 18.9 ± 0.2°, and 24.6 ± 0.2°;

X

In a certain embodiment, the X-ray powder diffraction (XRPD) pattern for the crystal form F, using Cu-Kα radiation and represented by 2θ angles, further comprises diffraction peaks at one or more of 9.8 ± 0.2°, 13.3 ± 0.2°, 17.5 ± 0.2°, and 20.8 ± 0.2°.

In a certain embodiment, the X-ray powder diffraction (XRPD) pattern for the crystal form F, using Cu-Kα radiation and represented by 2θ angles, has positions of diffraction peaks as shown in the following table:

| No. | Angle (°) | No. | Angle (°) |
|---|---|---|---|
| 1 | 5.8 | 14 | 17.5 |
| 2 | 6.6 | 15 | 18.3 |
| 3 | 7.2 | 16 | 18.9 |
| 4 | 9.8 | 17 | 20.0 |
| 5 | 10.1 | 18 | 20.8 |
| 6 | 11.2 | 19 | 21.7 |
| 7 | 12.2 | 20 | 22.5 |
| 8 | 13.3 | 21 | 24.1 |
| 9 | 13.6 | 22 | 24.6 |
| 10 | 14.5 | 23 | 26.5 |
| 11 | 15.7 | 24 | 27.0 |
| 12 | 16.2 | 25 | 28.9 |
| 13 | 16.6 | 26 | 35.5 |

In a certain embodiment, the X-ray powder diffraction (XRPD) pattern for the crystal form F, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 5.8 | 20.4% | 14 | 17.5 | 43.5% |
| 2 | 6.6 | 29.1% | 15 | 18.3 | 17.5% |

(continued)

| No. | Angle (°) | Relative intensity | No. | Angle (°) | Relative intensity |
|---|---|---|---|---|---|
| 3 | 7.2 | 60.6% | 16 | 18.9 | 39.1% |
| 4 | 9.8 | 8.9% | 17 | 20.0 | 24.5% |
| 5 | 10.1 | 6.2% | 18 | 20.8 | 38.9% |
| 6 | 11.2 | 15.6% | 19 | 21.7 | 17.6% |
| 7 | 12.2 | 56.5% | 20 | 22.5 | 48.3% |
| 8 | 13.3 | 100.0% | 21 | 24.1 | 18.1% |
| 9 | 13.6 | 37.6% | 22 | 24.6 | 21.4% |
| 10 | 14.5 | 28.2% | 23 | 26.5 | 9.4% |
| 11 | 15.7 | 74.0% | 24 | 27.0 | 10.4% |
| 12 | 16.2 | 30.9% | 25 | 28.9 | 3.4% |
| 13 | 16.6 | 13.9% | 26 | 35.5 | 3.3% |

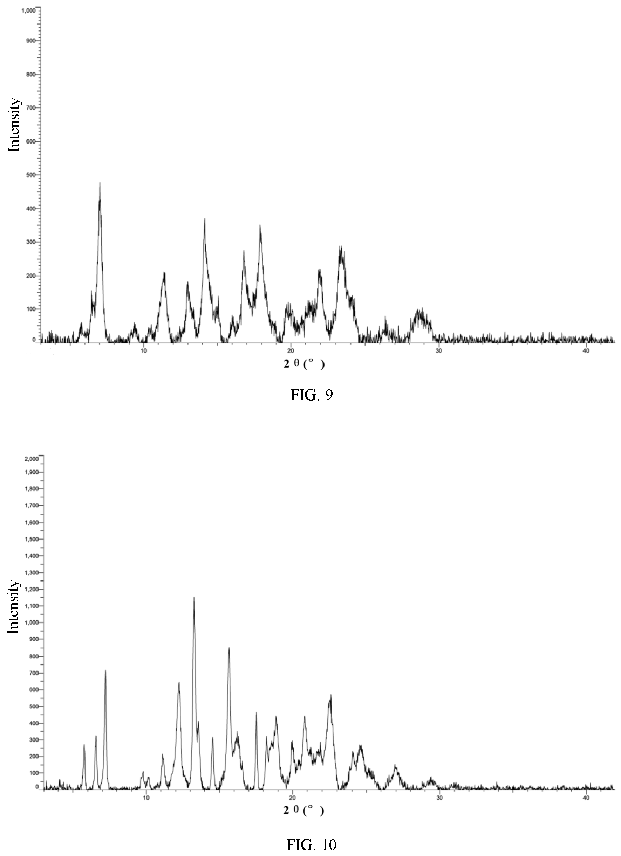

In a certain embodiment, the X-ray powder diffraction (XRPD) pattern for the crystal form F, using Cu-K$\alpha$ radiation and represented by 2$\theta$ angles, is substantially as shown in FIG. 10.

In a certain embodiment, the crystal form F has a differential scanning calorimetry (DSC) pattern comprising endothermic peaks at 249.1 $\pm$ 5°C and 262.9 $\pm$ 5°C and an exothermic peak at 258.6 $\pm$ 5°C.

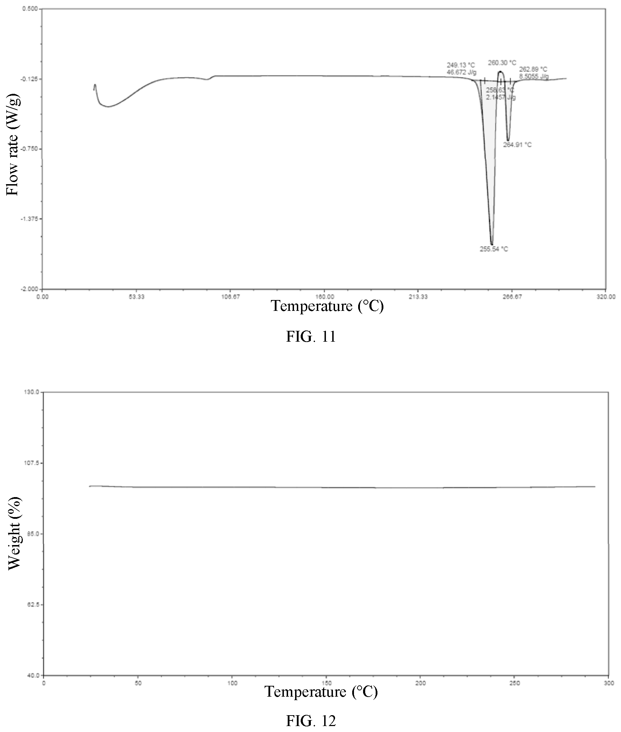

In a certain embodiment, the differential scanning calorimetry (DSC) pattern for the crystal form F is substantially as shown in FIG. 11.

In a certain embodiment, the crystal form F has a thermogravimetric analysis (TGA) pattern with essentially no weight loss before 300 $\pm$ 5°C.

In a certain embodiment, the thermogravimetric analysis (TGA) pattern for the crystal form F is substantially as shown in FIG. 12.

The present disclosure further provides a crystal form H of the five-membered-fused six-membered compound of formula X, which has an X-ray powder diffraction (XRPD) pattern using Cu-K$\alpha$ radiation and represented by 2$\theta$ angles comprising diffraction peaks at 5.8 $\pm$ 0.2°, 8.5 $\pm$ 0.2°, 9.5 $\pm$ 0.2°, 11.7 $\pm$ 0.2°, 14.1 $\pm$ 0.2°, 16.9 $\pm$ 0.2°, and 20.8 $\pm$ 0.2°;

X .

In a certain embodiment, the X-ray powder diffraction (XRPD) pattern for the crystal form H, using Cu-K$\alpha$ radiation and represented by 2$\theta$ angles, further comprises diffraction peaks at one or more of 10.9 $\pm$ 0.2°, 19.1 $\pm$ 0.2°, 26.5 $\pm$ 0.2°, and 33.0 $\pm$ 0.2°.

In a certain embodiment, the X-ray powder diffraction (XRPD) pattern for the crystal form H, using Cu-K$\alpha$ radiation and represented by 2$\theta$ angles, has positions of diffraction peaks as shown in the following table:

| No. | Angle (°) | No. | Angle (°) |
|---|---|---|---|
| 1 | 5.8 | 17 | 20.8 |
| 2 | 8.5 | 18 | 21.8 |
| 3 | 9.5 | 19 | 22.5 |
| 4 | 10.9 | 20 | 23.0 |

(continued)

| No. | Angle (°) | No. | Angle (°) |
|---|---|---|---|
| 5 | 11.7 | 21 | 23.3 |
| 6 | 14.1 | 22 | 23.9 |
| 7 | 14.4 | 23 | 24.5 |
| 8 | 14.9 | 24 | 25.4 |
| 9 | 15.5 | 25 | 25.8 |
| 10 | 15.9 | 26 | 26.5 |
| 11 | 16.6 | 27 | 27.4 |
| 12 | 16.9 | 28 | 28.0 |
| 13 | 17.2 | 29 | 28.2 |
| 14 | 17.6 | 30 | 32.4 |
| 15 | 18.5 | 31 | 33.0 |
| 16 | 19.1 | | |

In a certain embodiment, the X-ray powder diffraction (XRPD) pattern for the crystal form H, using Cu-K$\alpha$ radiation and represented by 2$\theta$ angles, has positions and relative intensities of diffraction peaks as shown in the following table:

| No. | Angle (°) | Relative intensity | No. | Angle (°) | Relative intensity |
|---|---|---|---|---|---|
| 1 | 5.8 | 5.7% | 17 | 20.8 | 100.0% |
| 2 | 8.5 | 18.7% | 18 | 21.8 | 8.1% |
| 3 | 9.5 | 9.6% | 19 | 22.5 | 3.4% |
| 4 | 10.9 | 37.0% | 20 | 23.0 | 6.1% |
| 5 | 11.7 | 3.7% | 21 | 23.3 | 4.8% |
| 6 | 14.1 | 12.0% | 22 | 23.9 | 6.7% |
| 7 | 14.4 | 3.7% | 23 | 24.5 | 6.0% |
| 8 | 14.9 | 2.6% | 24 | 25.4 | 7.5% |
| 9 | 15.5 | 8.2% | 25 | 25.8 | 42.5% |
| 10 | 15.9 | 9.7% | 26 | 26.5 | 11.0% |
| 11 | 16.6 | 7.9% | 27 | 27.4 | 3.6% |
| 12 | 16.9 | 85.1% | 28 | 28.0 | 3.9% |
| 13 | 17.2 | 32.5% | 29 | 28.2 | 4.8% |
| 14 | 17.6 | 15.6% | 30 | 32.4 | 3.1% |
| 15 | 18.5 | 17.0% | 31 | 33.0 | 3.3% |
| 16 . | 19.1 | 28.2% | | | |

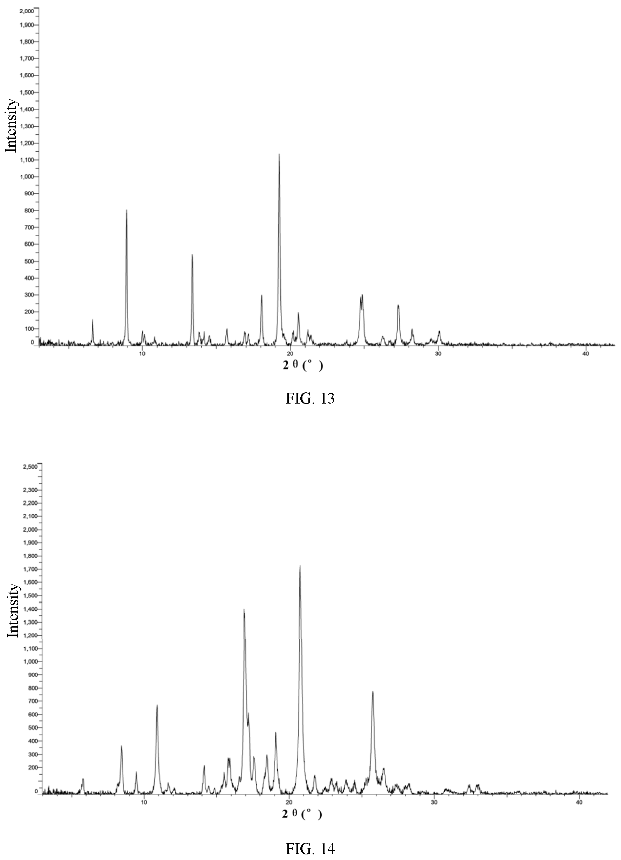

In a certain embodiment, the X-ray powder diffraction (XRPD) pattern for the crystal form H, using Cu-K$\alpha$ radiation and represented by 2$\theta$ angles, is substantially as shown in FIG. 14.

In a certain embodiment, the crystal form H has a differential scanning calorimetry (DSC) pattern comprising an endothermic peak at 288.5 $\pm$ 5°C.

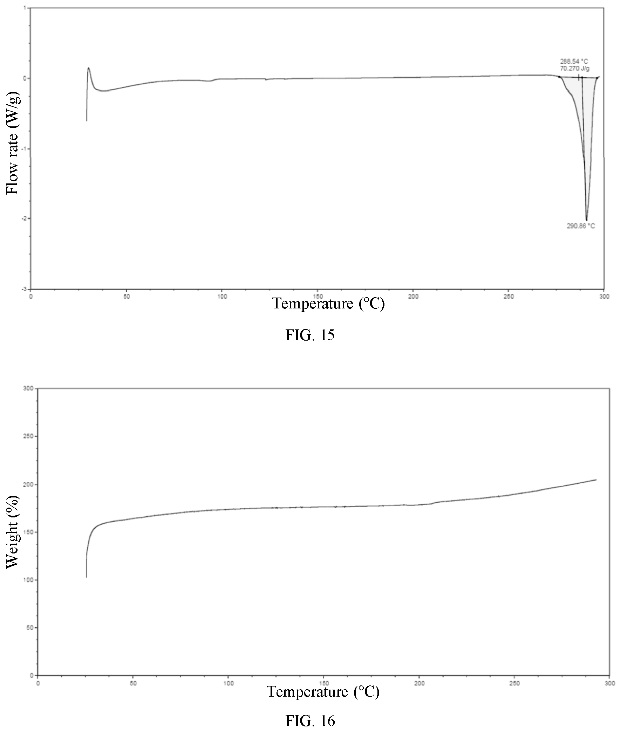

In a certain embodiment, the differential scanning calorimetry (DSC) pattern for the crystal form H is substantially as shown in FIG. 15.

In a certain embodiment, the crystal form H has a thermogravimetric analysis (TGA) pattern with essentially no

weight loss before 300 ± 5°C.

In a certain embodiment, the thermogravimetric analysis (TGA) pattern for the crystal form H is substantially as shown in FIG. 16.

The present disclosure further provides a crystal form I of the five-membered-fused six-membered compound of formula X, which has an X-ray powder diffraction (XRPD) pattern using Cu-Kα radiation and represented by 2θ angles comprising diffraction peaks at 8.0 ± 0.2°, 11.3 ± 0.2°, 15.5 ± 0.2°, 17.6 ± 0.2°, 20.2 ± 0.2°, and 25.6 ± 0.2°;

X

.

In a certain embodiment, the X-ray powder diffraction (XRPD) pattern for the crystal form I, using Cu-Kα radiation and represented by 2θ angles, further comprises diffraction peaks at one or more of 7.6 ± 0.2°, 18.6 ± 0.2°, 22.0 ± 0.2°, and 31.7 ± 0.2°.

In a certain embodiment, the X-ray powder diffraction (XRPD) pattern for the crystal form I, using Cu-Kα radiation and represented by 2θ angles, has positions of diffraction peaks as shown in the following table:

| No. | Angle (°) | No. | Angle (°) |
|---|---|---|---|
| 1 | 7.6 | 12 | 21.1 |
| 2 | 8.0 | 13 | 21.5 |
| 3 | 11.3 | 14 | 22.0 |
| 4 | 12.1 | 15 | 22.8 |
| 5 | 15.5 | 16 | 23.2 |
| 6 | 16.0 | 17 | 24.2 |
| 7 | 16.5 | 18 | 25.3 |
| 8 | 17.0 | 19 | 25.6 |
| 9 | 17.6 | 20 | 26.8 |
| 10 | 18.6 | 21 | 28.5 |
| 11 | 20.2 | 22 | 31.7 |

In a certain embodiment, the X-ray powder diffraction (XRPD) pattern for the crystal form I, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 7.6 | 11.0% | 12 | 21.1 | 11.3% |
| 2 | 8.0 | 41.7% | 13 | 21.5 | 10.9% |
| 3 | 11.3 | 100.0% | 14 | 22.0 | 49.1% |
| 4 | 12.1 | 81.3% | 15 | 22.8 | 17.7% |
| 5 | 15.5 | 21.0% | 16 | 23.2 | 11.2% |
| 6 | 16.0 | 31.3% | 17 | 24.2 | 15.3% |
| 7 | 16.5 | 28.7% | 18 | 25.3 | 49.8% |

(continued)

| No. | Angle (°) | Relative intensity | No. | Angle (°) | Relative intensity |
|---|---|---|---|---|---|
| 8 | 17.0 | 13.0% | 19 | 25.6 | 21.0% |
| 9 | 17.6 | 47.4% | 20 | 26.8 | 12.0% |
| 10 | 18.6 | 18.4% | 21 | 28.5 | 12.6% |
| 11 | 20.2 | 51.3 | 22 | 31.7 | 4.9% |

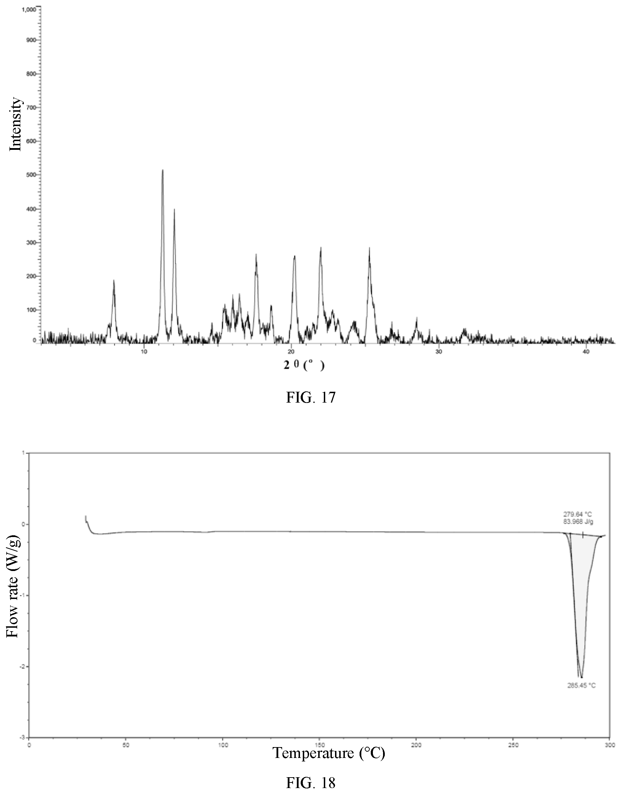

In a certain embodiment, the X-ray powder diffraction (XRPD) pattern for the crystal form I, using Cu-K$\alpha$ radiation and represented by 2$\theta$ angles, is substantially as shown in FIG. 17.

In a certain embodiment, the crystal form I has a differential scanning calorimetry (DSC) pattern comprising an endothermic peak at 279.6 $\pm$ 5°C.

In a certain embodiment, the differential scanning calorimetry (DSC) pattern for the crystal form I is substantially as shown in FIG. 18.

In a certain embodiment, the crystal form I has a thermogravimetric analysis (TGA) pattern with a weight loss of 0.865% before 150 $\pm$ 5°C.

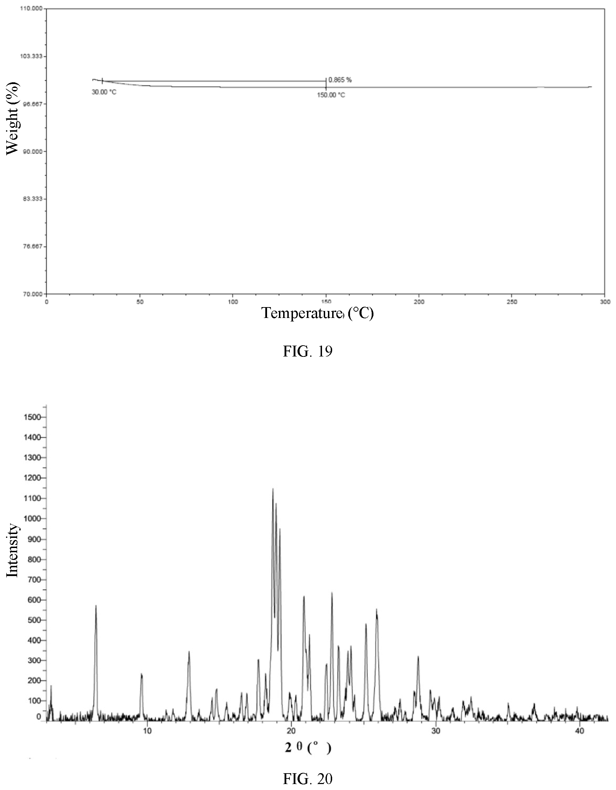

In a certain embodiment, the thermogravimetric analysis (TGA) pattern for the crystal form I is substantially as shown in FIG. 19.

The present disclosure further provides a crystal form B of the five-membered-fused six-membered compound of formula X, which has an X-ray powder diffraction (XRPD) pattern using Cu-K$\alpha$ radiation and represented by 2$\theta$ angles comprising diffraction peaks at 8.6 $\pm$ 0.2°, 11.8 $\pm$ 0.2°, 15.8 $\pm$ 0.2°, and 18.9 $\pm$ 0.2°;

X

In a certain embodiment, the X-ray powder diffraction (XRPD) pattern for the crystal form B, using Cu-K$\alpha$ radiation and represented by 2$\theta$ angles, further comprises diffraction peaks at 17.3 $\pm$ 0.2° and/or 25.9 $\pm$ 0.2°.

In a certain embodiment, the X-ray powder diffraction (XRPD) pattern for the crystal form B, using Cu-K$\alpha$ radiation and represented by 2$\theta$ angles, has positions and relative intensities of diffraction peaks as shown in the following table:

| No. | Angle (°) | Relative intensity | No. | Angle (°) | Relative intensity |
|---|---|---|---|---|---|
| 1 | 8.6 | 83.1% | 9 | 17.3 | 39.7% |
| 2 | 9.5 | 17.3% | 10 | 18.4 | 58.7% |
| 3 | 11.6 | 15.4% | 11 | 18.9 | 100.0% |
| 4 | 11.8 | 15.7% | 12 | 24.1 | 18.4% |
| 5 | 13.4 | 22.3% | 13 | 24.6 | 20.2% |
| 6 | 13.6 | 14.1% | 14 | 25.9 | 26.4% |
| 7 | 15.5 | 16.0% | 15 | 27.0 | 13.9% |
| 8 . | 15.8 | 14.7% | 16 | 33.2 | 14.4% |

In a certain embodiment, the X-ray powder diffraction (XRPD) pattern for the crystal form B, using Cu-K$\alpha$ radiation and represented by 2$\theta$ angles, is substantially as shown in FIG. 5.

The present disclosure further provides a crystal form D of the five-membered-fused six-membered compound of

formula X, which has an X-ray powder diffraction (XRPD) pattern using Cu-Kα radiation and represented by 2θ angles comprising diffraction peaks at 6.5 ± 0.2°, 7.0 ± 0.2°, 11.4 ± 0.2°, 13.0 ± 0.2°, 17.9 ± 0.2°, and 22.0 ± 0.2°;

X

.

In a certain embodiment, the X-ray powder diffraction (XRPD) pattern for the crystal form D, using Cu-Kα radiation and represented by 2θ angles, further comprises diffraction peaks at one or more of 10.4 ± 0.2°, 16.8 ± 0.2°, and 26.4 ± 0.2°.

In a certain embodiment, the X-ray powder diffraction (XRPD) pattern for the crystal form D, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.5 | 27.2% | 9 | 16.8 | 61.9% |
| 2 | 7.0 | 100.0% | 10 | 17.9 | 75.9% |
| 3 | 10.4 | 9.2% | 11 | 18.8 | 10.5% |
| 4 | 11.4 | 48.6% | 12 | 19.7 | 25.4% |
| 5 | 13.0 | 34.8% | 13 | 22.0 | 47.2% |
| 6 | 14.1 | 81.1% | 14 | 23.4 | 66.2% |
| 7 | 15.0 | 22.8% | 15 | 24.1 | 32.1% |
| 8 | 16.1 | 17.8% | 16 | 26.4 | 13.9% |

In a certain embodiment, the X-ray powder diffraction (XRPD) pattern for the crystal form D, using Cu-Kα radiation and represented by 2θ angles, is substantially as shown in FIG. 9.

The present disclosure further provides a crystal form G of the five-membered-fused six-membered compound of formula X, which has an X-ray powder diffraction (XRPD) pattern using Cu-Kα radiation and represented by 2θ angles comprising diffraction peaks at 9.0 ± 0.2°, 13.4 ± 0.2°, 14.5 ± 0.2°, 19.3 ± 0.2°, and 24.8 ± 0.2°;

X

.

In a certain embodiment, the X-ray powder diffraction (XRPD) pattern for the crystal form G, using Cu-Kα radiation and represented by 2θ angles, further comprises diffraction peaks at one or more of 16.9 ± 0.2°, 20.6 ± 0.2°, and 27.3 ± 0.2°.

In a certain embodiment, the X-ray powder diffraction (XRPD) pattern for the crystal form G, using Cu-Kα

radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.6 | 12.1% | 11 | 19.3 | 100.0% |
| 2 | 9.0 | 66.3% | 12 | 20.2 | 6.4% |
| 3 | 13.4 | 46.8% | 13 | 20.6 | 17.7% |
| 4 | 13.9 | 5.6% | 14 | 21.2 | 8.0% |
| 5 | 14.2 | 7.3% | 15 | 24.8 | 16.2% |
| 6 | 14.5 | 4.9% | 16 | 26.2 | 4.6% |
| 7 | 15.7 | 9.8% | 17 | 26.7 | 2.0% |
| 8 | 16.9 | 4.9% | 18 | 27.3 | 22.0% |
| 9 | 17.2 | 5.9% | 19 | 28.2 | 10.4% |
| 10 | 18.1 | 27.2% | 20 | 30.1 | 6.9% |

In a certain embodiment, the X-ray powder diffraction (XRPD) pattern for the crystal form G, using Cu-Kα radiation and represented by 2θ angles, is substantially as shown in FIG. 13.

The present disclosure further provides a crystal form J of the five-membered-fused six-membered compound of formula X, which has an X-ray powder diffraction (XRPD) pattern using Cu-Kα radiation and represented by 2θ angles comprising diffraction peaks at 6.4 ± 0.2°, 9.6 ± 0.2°, 12.9 ± 0.2°, 16.5 ± 0.2°, 17.7 ± 0.2°, and 22.8 ± 0.2°;

X

In a certain embodiment, the X-ray powder diffraction (XRPD) pattern for the crystal form J, using Cu-Kα radiation and represented by 2θ angles, further comprises diffraction peaks at one or more of 16.9 ± 0.2°, 20.9 ± 0.2°, 25.9 ± 0.2°, and 28.8 ± 0.2°.

In a certain embodiment, the X-ray powder diffraction (XRPD) pattern for the crystal form J, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.4 | 50.3% | 17 | 21.3 | 36.8% |
| 2 | 9.6 | 18.9% | 18 | 22.4 | 24.3% |
| 3 | 12.9 | 30.5% | 19 | 22.8 | 55.0% |
| 4 | 14.5 | 9.1% | 20 | 23.3 | 32.5% |
| 5 | 14.8 | 13.7% | 21 | 23.9 | 30.4% |
| 6 | 15.5 | 7.8% | 22 | 24.1 | 32.5% |
| 7 | 16.5 | 11.6% | 23 | 25.2 | 42.1% |
| 8 | 16.9 | 12.0% | 24 | 25.9 | 45.9% |
| 9. | 17.7 | 26.4% | 25 | 27.5 | 8.8% |

(continued)

| No. | Angle (°) | Relative intensity | No. | Angle (°) | Relative intensity |
|---|---|---|---|---|---|
| 10 | 18.2 | 20.5% | 26 | 28.8 | 28.2% |
| 11 | 18.7 | 100.0% | 27 | 29.6 | 12.1% |
| 12 | 19.0 | 94.6% | 28 | 30.2 | 10.4% |
| 13 | 19.2 | 82.5% | 29 | 31.9 | 7.3% |
| 14 | 19.9 | 11.5% | 30 | 35.1 | 6.2% |
| 15 | 20.3 | 11.0% | 31 | 36.8 | 6.3% |
| 16 | 20.9 | 57.0% | | | |

In a certain embodiment, the X-ray powder diffraction (XRPD) pattern for the crystal form J, using Cu-Kα radiation and represented by 2θ angles, is substantially as shown in FIG. 20.

The present disclosure further provides a hydrochloride or sulfate of the five-membered-fused six-membered compound of formula X,

**X**

.

The hydrochloride of the five-membered-fused six-membered compound of formula X can be as shown in formula X-1,

· 0.8HCl

**X-1**

.

The sulfate of the five-membered-fused six-membered compound of formula X can be as shown in formula X-2,

· $0.5H_2SO_4$

**X-2**

.

The present disclosure further provides a preparation method for the crystal form A of the five-membered-fused six-membered compound of formula X as described above, comprising the step of: slurrying the five-membered-fused six-membered compound of formula X in a ketone solvent to obtain the crystal form A. The ketone solvent is, for example, acetone. The five-membered-fused six-membered compound of formula X can be in an amorphous form.

The present disclosure further provides a preparation method for the crystal form C of the five-membered-fused six-membered compound of formula X as described above, comprising the step of: slurrying the five-membered-fused six-membered compound of formula X in a nitrile solvent to obtain the crystal form C. The nitrile solvent is, for example, acetonitrile. The five-membered-fused six-membered compound of formula X can be in an amorphous form.

The present disclosure further provides a preparation method for the crystal form F of the five-membered-fused six-membered compound of formula X as described above, comprising the step of: subjecting the five-membered-fused six-membered compound of formula X and a chloroalkane to gas-solid diffusion to obtain the crystal form F. The chloroalkane is, for example, dichloromethane. The five-membered-fused six-membered compound of formula X can be in an amorphous form.

The present disclosure further provides a preparation method for the crystal form H of the five-membered-fused six-membered compound of formula X as described above, comprising the step of: adding a benzene hydrocarbon solvent to a solution of the five-membered-fused six-membered compound of formula X in an amide solvent for crystallization to obtain the crystal form H. The amide solvent is, for example, DMF. The benzene hydrocarbon solvent is, for example, toluene.

The five-membered-fused six-membered compound of formula X and the amide solvent can have a mass-to-volume ratio of 80 to 150 mg/mL, preferably 100 to 125 mg/mL, for example, 100 mg/mL or 125 mg/mL.

The benzene hydrocarbon solvent and the amide solvent can have a volume ratio of (5 to 15):1, preferably (8 to 12):1, for example, 10:1.

The crystallization can be performed at room temperature.

The present disclosure further provides a preparation method for the crystal form I of the five-membered-fused six-membered compound of formula X as described above, comprising the step of: adding an ether solvent to a solution of the five-membered-fused six-membered compound of formula X in a sulfoxide solvent for crystallization to obtain the crystal form I. The sulfoxide solvent is, for example, DMSO. The ether solvent is, for example, MTBE.

The five-membered-fused six-membered compound of formula X and the sulfoxide solvent can have a mass-to-volume ratio of 80 to 150 mg/mL, preferably 100 to 125 mg/mL, for example, 100 mg/mL or 125 mg/mL.

The ether solvent and the sulfoxide solvent can have a volume ratio of (5 to 15):1, preferably (8 to 12):1, for example, 10:1.

The crystallization can be performed at room temperature.

The present disclosure further provides a pharmaceutical composition, comprising: (1) the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X; and

(2) a pharmaceutically acceptable carrier.

The present disclosure further provides a use of substance Z in the manufacture of an IRAK4 degrading agent, wherein the substance Z is the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X, or the pharmaceutical composition.

The present disclosure further provides a use of substance Z in the manufacture of a medicament for the treatment and/or prevention of a Myd88- and/or IRAK4-related disease; the substance Z is the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X, or the pharmaceutical composition.

In a certain embodiment, the IRAK4-related disease comprises one or more of a chronic lung disease, an autoimmune disease, an inflammatory disease, a tumor, a cardiovascular and cerebrovascular disease, and a central nervous system disease.

In a certain embodiment, the autoimmune disease comprises psoriasis and rheumatoid arthritis.

In a certain embodiment, the autoimmune disease comprises psoriasis, systemic lupus erythematosus, and rheumatoid arthritis.

In a certain embodiment, the inflammatory disease comprises ulcerative colitis.

In a certain embodiment, the inflammatory disease comprises an inflammatory bowel disease, such as ulcerative colitis.

In a certain embodiment, the tumor can be a hematological tumor or a solid tumor.

In a certain embodiment, the hematological tumor comprises large B-cell lymphoma and acute and chronic

lymphocytic leukemia.

In a certain embodiment, the solid tumor comprises intestinal cancer and skin cancer caused by MYD88 mutations.

In a certain embodiment, the cardiovascular and cerebrovascular disease comprises stroke and atherosclerosis.

In a certain embodiment, the central nervous system disease comprises primary central nervous system lymphoma.

The present disclosure further provides a use of substance Z in the manufacture of a medicament for the treatment of one or more of a chronic lung disease, an autoimmune disease, an inflammatory disease, a tumor, a cardiovascular and cerebrovascular disease, and a central nervous system disease; the substance Z is the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X, or the pharmaceutical composition.

The present disclosure further provides a method for treating and/or preventing a Myd88- and/or IRAK4-related disease, comprising administering an effective amount of substance Z to a patient, wherein the substance Z is the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X, or the pharmaceutical composition.

The present disclosure further provides substance Z for the treatment and/or prevention of a Myd88- and/or IRAK4-related disease, wherein the substance Z is the crystal form A, crystal form B, crystal form C, crystal form D, crystal form F, crystal form G, crystal form H, crystal form I, or crystal form J of the five-membered-fused six-membered compound of formula X, or the hydrochloride or sulfate of the five-membered-fused six-membered compound of formula X, or the pharmaceutical composition.

The crystal forms of the present disclosure can be characterized by one or more solid-state analytical methods. For example, X-ray powder diffraction, single crystal X-ray diffraction, differential scanning calorimetry, thermogravimetric analysis. Those skilled in the art know that the peak intensity and/or peak situation of X-ray powder diffraction may vary due to different experimental conditions. At the same time, the measured 2θ value will have an error of about ± 0.20° due to different accuracy of the instrument. The relative intensityvalue of the peak is more dependent on some properties of the sample being measured, such as crystal size and purity, compared to the peak position. Therefore, the measured peak intensity may show a deviation of about ± 0.2. Despite experimental errors, instrumental errors, and preferential orientation, those skilled in the art can also obtain sufficient information for identifying crystal forms from the X-ray powder diffraction data provided in the present patent. In DSC measurement, the initial temperature, the maximum temperature, and the heat of fusion data of the endothermic peak obtained by actual measurement are variable to a certain extent according to the heating rate, crystal shape and purity, and other measurement parameters.

In the present disclosure, "room temperature" means 15 to 35°C.

In the present disclosure, "gas-solid diffusion" means that a compound solid is placed in one container, a solvent is placed in another container, and the former is placed in the latter in an open state and allowed to stand in a sealed state.

Explanation of terms:

The term "substantially" means that the position of each peak in the figure can vary slightly with slight variations in measurement equipment, measurement conditions, and between batches of the product to be measured, and is not considered to be an absolute value.

The term "treatment" refers to therapeutic therapy. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder.

The term "prevention" refers to the reduction of the risk of acquiring or developing a disease or disorder.

On the basis of not violating common sense in the art, the above preferred conditions can be combined arbitrarily to obtain preferred examples of the present disclosure.

The reagents and starting materials used in the present disclosure are all commercially available.

The positive and progressive effect of the present disclosure lies in that the present disclosure discloses a crystal form of a five-membered-fused six-membered compound, a preparation method therefor, and a use thereof. The crystal form of the five-membered-fused six-membered compound of the present disclosure has an inhibitory or/and degrading effect on IRAK4 with potential clinical application value, and is expected to improve the prognosis of patients and reduce the possibility of drug resistance. Moreover, it also has good solubility, physicochemical stability, and mechanical stability.

BRIEF DESCRIPTION OF THE DRAWINGS

FIG. 1 shows an XRPD pattern of compound X in an amorphous form.

FIG. 2 shows an XRPD pattern of crystal form A.

FIG. 3 shows a DSC pattern of crystal form A.

FIG. 4 shows a TGA pattern of crystal form A.

FIG. 5 shows an XRPD pattern of crystal form B.

FIG. 6 shows an XRPD pattern of crystal form C.

FIG. 7 shows a DSC pattern of crystal form C.

FIG. 8 shows a TGA pattern of crystal form C.

FIG. 9 shows an XRPD pattern of crystal form D.

FIG. 10 shows an XRPD pattern of crystal form F.

FIG. 11 shows a DSC pattern of crystal form F.

FIG. 12 shows a TGA pattern of crystal form F.

FIG. 13 shows an XRPD pattern of crystal form G.

FIG. 14 shows an XRPD pattern of crystal form H.

FIG. 15 shows a DSC pattern of crystal form H.

FIG. 16 shows a TGA pattern of crystal form H.

FIG. 17 shows an XRPD pattern of crystal form I.

FIG. 18 shows a DSC pattern of crystal form I.

FIG. 19 shows a TGA pattern of crystal form I.

FIG. 20 shows an XRPD pattern of crystal form J.

FIG. 21 shows a graph of adsorption isotherms determined by DVS for crystal form I.

FIG. 22 shows a graph of dynamic adsorption determined by DVS for crystal form I.

FIG. 23 shows a graph of adsorption isotherms determined by DVS for crystal form A.

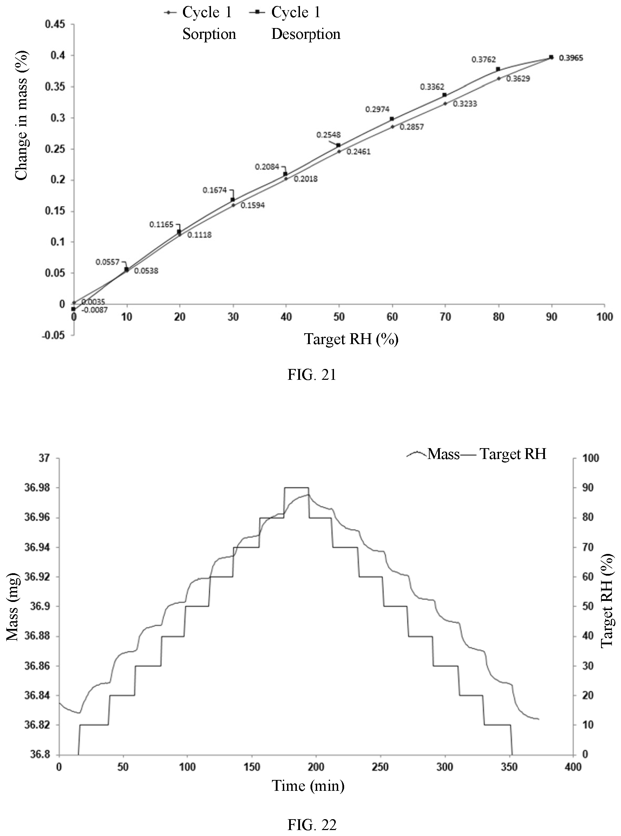

FIG. 22 shows a graph of dynamic adsorption determined by DVS for crystal form A.

DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

Abbreviation:

| Abbreviation | Name in Chinese | Abbreviation | Name in Chinese |
|---|---|---|---|
| ACN | Acetonitrile | FaSSIF | Fasted state simulated intestinal fluid |
| DCM | Dichloromethane | FeSSIF | Fed state simulated intestinal fluid |
| DMF | Dimethylformamide | MeOH | Methanol |
| DMSO | Dimethyl sulfoxide | MTBE | Methyl *tert*-butyl ether |
| EtOAc | Ethyl acetate | SGF | Simulated gastric fluid |
| EtOH | Ethanol | TFA | Trifluoroacetic acid |

Instruments and methods:

1. X-ray powder diffraction (XRPD)

Solid samples are analyzed using a Bruker D8 ADVANCE X-ray powder diffractometer (Bruker, Germany) for diffraction data collection at room temperature. The X-ray source is Cu Kα (λ = 1.5406 Å, tube voltage and current: 40 kV and 40 mA). During sample preparation, an appropriate amount of sample is placed on a zero-background sample holder, and the surface is gently pressed flat. Samples are scanned from 3° to 42° (2θ) with a step size of 0.02° (2θ) and a scan time of 0.05 seconds per step.

2. Differential scanning calorimetry (DSC)

Samples are subjected to DSC analysis using Discovery DSC25 (TA Instruments, USA) equipped with an RCS90 cooling system. 0.5 to 5 mg of sample is placed in a perforated aluminum pan and heated from 25°C to the endpoint temperature at a rate of 10°C/min under a nitrogen atmosphere with a flow rate of 50 mL/min. Measurements are performed under a nitrogen atmosphere with a constant flow rate of 50 mL/min.

3. Thermogravimetric analysis (TGA)

Samples are subjected to thermogravimetric analysis using Discovery TGA550 (TA Instruments, USA). 1 to 5 mg of sample is placed in a tared alumina pan and heated from room temperature to the endpoint temperature at a rate of 10°C/min under a nitrogen atmosphere with a flow rate of 60 mL/min.

4. Proton nuclear magnetic resonance (1H-NMR)

Samples are subjected to NMR analysis by characterization of crystal forms using a Varian Mercury Plus 400 MHz NMR spectrometer (Varian, USA), with deuterated dimethyl sulfoxide used as a solvent.

5. Dynamic vapor sorption (DVS)

Samples are analyzed for hygroscopicity using Intrinsic DVS (System Measurement System, UK). Approximately 10 to 30 mg of sample is used for testing. The testing chamber is controlled at a temperature of 25 ± 1°C with the specific testing parameters shown below:

| | |
|---|---|
| **dm/dt** | 0.002%/min |
| **Cycle** | Full cycle |
| **Atmosphere and flow rate** | $N_2$, 200 mL/min |
| **Data retention rate** | 20 s |
| **Minimum equilibrium time for dm/dt** | 10 min |
| **Maximum equilibrium time** | 120 min |

(continued)

| | |
|---|---|
| **RH range** | 0% RH to 90% RH to 0% RH |
| **RH gradient** | 10% |

6. Ion chromatography (IC)

Samples are analyzed using a Dionex ICS-1500 ion chromatograph with the following parameters:

| | | |
|---|---|---|
| **Chromatographic column** | Dionex IonPac AS22 Analytical (4 × 250 mm) | |
| | Dionex IonPac AG22 Guard (4 × 50 mm) | |
| **Flow rate** | 1.3 mL/min | |
| **Column temperature** | 30°C | |
| **Injection volume** | 25 μL | |
| **Elution time (RT)** | Compound | RT (min) |
| | Chloride | 4.3 |
| | Nitrite | 5.2 |
| | Bromide | 6.3 |
| | Nitrate | 7.1 |
| | Phosphate | 9.8 |
| | Sulfate | 11.1 |
| | Iodide | 11.1 |
| **Run time** | 16 minutes | |
| **Suppressor current** | 35 mA | |
| **Eluent** | 4.8 mM $NaCO_3$: 1.0 mM $NaHCO_3$ | |

Example 1: Synthesis of compound X

Step 1: Synthesis of Int-A1

2-(2,6-Dioxopiperidin-3-yl)-5-fluoroisoindoline-1,3-dione (5.0 g, 18.0 mmol) was dissolved in anhydrous dimethyl sulfoxide (20.0 mL), followed by the addition of DIPEA (*N,N*-diisopropylethylamine, 4.7 g, 36.2 mmol) and *tert*-butyl piperidine-4-carboxylate (4.0 g, 21.6 mmol), and the reaction mixture was stirred in an oil bath at 90°C for 2 hours until the reaction was complete. The reaction system was cooled to room temperature, diluted with water, and extracted with EA (ethyl acetate, 50 mL). The phases were separated, and the aqueous phase was extracted with EA (50 mL × 3). The combined organic phases were washed with brine (50 mL), dried over magnesium sulfate, filtered, and concentrated in vacuum to obtain a crude product. The crude product was purified by column chromatography (PE/EA = 5/1) to obtain product Int-A1 (7.5 g, yield: 94.2%) as a yellow solid, MS (ESI) m/z: 442.2 $[M+H]^+$.

Step 2: Synthesis of Int-A

TFA (trifluoroacetic acid, 20 mL) was added dropwise to a mixture of Int-A1 (5 g, 11.3 mmol) and DCM (dichloromethane, 50 mL) at 0°C. After the dropwise addition was completed, the reaction mixture was stirred at 25°C for 16 hours until the reaction was complete. The reaction mixture was washed three times with ether to remove the residual TFA from the reaction mixture to obtain product Int-A (3.7 g, yield: 84.9%) as a yellow solid, MS (ESI) m/z: 386.1 $[M+H]^+$.

Step 3: Synthesis of I-1-A

A mixture of 7-*tert*-butoxycarbonyl-7-azaspiro[3.5]-2-nonanol (600 mg, 2.49 mmol), 4-dimethylaminopyridine (60.75 mg, 497.25 μmol, 83.68 μL), triethylamine (503.17 mg, 4.97 mmol, 693.07 μL), and dichloromethane (1.35 mL) was dissolved under stirring, then *p*-toluenesulfonyl chloride (521.40 mg, 2.73 mmol) was added thereto, and the mixture was heated to 40°C and stirred for 18 hours until the reaction was complete. The reaction system was diluted with water (30 mL) and extracted with dichloromethane (20 mL × 3) under stirring. The combined organic phases were washed with brine (20 mL), dried over anhydrous sodium sulfate, filtered, and concentrated in vacuum to obtain I-1-A (1 g, crude product), MS (ESI) m/z: 396.2 $[M+H]^+$.

Step 4: Synthesis of I-1-1

A mixture of 6-methoxy-5-nitro-2*H*-indazole (2 g, 10.35 mmol), Pd/C (0.3 g, purity: 10%), and methanol (30 mL) was stirred and replaced with hydrogen three times. The mixture was then stirred under a hydrogen atmosphere at 25°C for 18 hours until the reaction was complete. The mixture was filtered through diatomite to remove the catalyst, and the filter cake was washed with methanol (10 mL × 2). The filtrate was concentrated under reduced pressure to obtain product I-1-1

(1.59 g, crude product) as a brown solid, MS (ESI) m/z: 164.1 $[M+H]^+$, which was directly used in the next step.

Step 5: Synthesis of I-1-2

Propylphosphonic anhydride (7.44 g, 11.69 mmol, purity: 50%) was added dropwise to a mixture of I-1-1 (1.59 g, 9.74 mmol), 6-(trifluoromethyl)pyridine-2-carboxylic acid (2.05 g, 10.72 mmol), diisopropylethylamine (3.78 g, 29.23 mmol, 5.09 mL), and tetrahydrofuran (25 mL) under stirring at 0°C. After the dropwise addition was completed, the reaction system was maintained under a nitrogen atmosphere at 15°C and stirred for 2 hours until the reaction was complete. The reaction mixture was added with water (50 mL) and extracted with ethyl acetate (40 mL × 5). The combined organic phases were washed with water (30 mL) and saturated brine, dried over anhydrous sodium sulfate, filtered, and concentrated in vacuum to obtain product I-1-2 (3.08 g, crude product) as a brown solid, MS (ESI) m/z: 337.1 $[M+H]^+$, which was directly used in the next step.

Step 6: Synthesis of I-1-3

A mixture of I-1-A (500 mg, 1.49 mmol), I-1-2 (705.71 mg, 1.78 mmol), cesium carbonate (968.93 mg, 2.97 mmol), and *N,N*-dimethylformamide (4 mL) was stirred at 90°C for 5 hours until the reaction was complete. The reaction mixture was cooled to room temperature, diluted with water (30 mL), and extracted with ethyl acetate (30 mL × 3) under stirring. The combined organic phases were washed with saturated brine (20 mL), dried over anhydrous sodium sulfate, filtered, and concentrated in vacuum to obtain a crude product, which was subjected to flash column chromatography (eluent PE/EA: 1/1) to obtain I-1-3 (250 mg, 446.76 μmol, yield: 30.05%), MS (ESI) m/z: 560.2 $[M+H]^+$.

Step 7: Synthesis of I-1-4

A mixture of I-1-3 (250 mg, 446.76 μmol), dichloromethane (3 mL), and trifluoroacetic acid (3 mL) was stirred at room temperature for 2 hours until the reaction was complete. The reaction mixture was rotary evaporated to dryness to obtain a crude product, which was purified by C18 column chromatography to obtain I-1-4 (200 mg, 435.29 μmol, yield: 97.43%) as a yellow solid, MS (ESI) m/z: 460.2 $[M+H]^+$.

Step 8: Synthesis of X

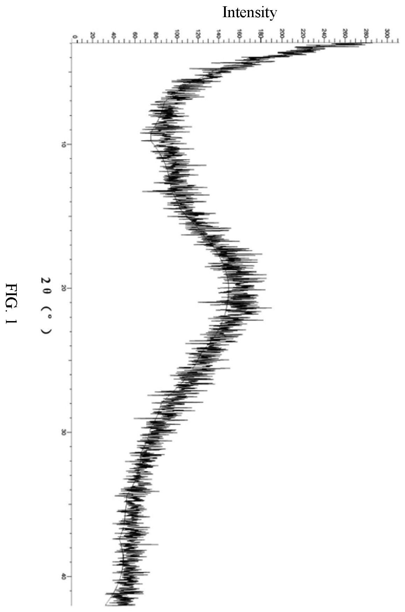

DIPEA (475 mg, 3.68 mmol) and I-1-4 (850 mg, 1.84 mmol) were added to a mixture of Int-A (709 mg, 1.84 mmol), HATU (840 mg, 2.21 mmol), and DMF (10 mL) at 0°C. The resulting reaction mixture was then stirred at 25°C for 1 hour until the reaction was complete. The reaction mixture was added with water (10 mL) and extracted with ethyl acetate (30 mL × 3). The combined organic phases were washed with brine, dried over sodium sulfate, filtered, and concentrated in vacuum to obtain a crude product. The crude product was purified by preparative HPLC to obtain product X (904 mg, yield: 59.4%) as a white solid. MS (ESI) m/z: 827.3 $[M+H]^+$; $^1H$ NMR (500 MHz, DMSO-$d_6$) δ 11.08 (s, 1H), 10.51 (s, 1H), 8.69 (s, 1H), 8.46 (d, *J* = 8.0 Hz, 1H), 8.41 (t, *J* = 8.0 Hz, 1H), 8.37 (s, 1H), 8.22 (d, *J* = 8.0 Hz, 1H), 7.67 (d, *J* = 8.5 Hz, 1H), 7.33 (s, 1H), 7.25 (d, *J* = 8.5 Hz, 1H), 7.21 (s, 1H), 5.24 - 5.12 (m, 1H), 5.07 (dd, *J* = 13.0, 5.0 Hz, 1H), 4.07 (d, *J* = 12.5 Hz, 2H), 3.99 (s, 3H), 3.60 - 3.38 (m, 4H), 3.15 - 3.05 (m, 2H), 3.04 - 2.95 (m, 1H), 2.94 - 2.83 (m, 1H), 2.66 - 2.52 (m, 2H), 2.50 - 2.46 (m, 2H), 2.46 - 2.38 (m, 2H), 2.06 - 1.98 (m, 1H), 1.81 - 1.67 (m, 4H), 1.67 - 1.54 (m, 4H). It was identified that compound X is in an amorphous form, whose XRPD is as shown in FIG. 1.

**Example 2: Application of compound X**

**2.1 Evaluation of inhibition of kinase activity by compound X**

Based on the experimental method of fluorescence microfluidic mobility assay, the IC50 value of competitive binding of IRAK4 kinase to ATP by the compound was determined. The initial detection concentration of the compound was 10 μM, which was 4-fold serially diluted to 0.38 nM and assayed in duplicate. In this case, commercially available staurosporine was the standard control for the assay.

2.1.1 Information on reagents and consumables is as follows:

| Name | Brand | Cat. No. |
|---|---|---|
| IRAK4 kinase | Carna | 09-145 |
| Substrate peptide FAM-P8 | GL Biochem | 112396 |

(continued)

| Name | Brand | Cat. No. |
|---|---|---|
| Adenosine 5'-triphosphate disodium salt hydrate | Merck | A7699-1G |
| Dimethyl sulfoxide (DMSO) | Merck | D2650 |
| Ethylene diamine tetraacetic acid (EDTA) | Merck | E5134 |
| Staurosporine | Selleckchem | S1421 |
| 4-(2-Hydroxyethyl)-1-piperazineethanesulfonic acid (HEPES) | Gibco | 15630-080 |
| Polyethylene oxide lauroyl ether (Brij-35 solution) | Merck | 9002-92-0 |
| 1,4-Dithiothreitol (DDT) | Merck | D0632-20G |
| 0.2% Coating Reagent #3 | PerkinElmer | 760050 |
| 96-well plate | Corning | 3365 |
| 384-well plate | Corning | 3573 |

2.1.2 Experimental method

1) IRAK4 kinase was dissolved in kinase buffer (50 mM HEPES pH 7.5, 10 mM MgCl2, 2 mM DTT, and 0.01% Brij-35) at a final concentration of 6 nM.

2) The substrate peptide FAM-P8 and ATP were dissolved in the above kinase buffer, and the final concentrations of the substrate peptide FAM-P8 and ATP for IRAK4 were 3 μM and 10 μM, respectively.

3) Dilution of compound: the compound was first diluted to 50 μM, and then 4-fold serially diluted with DMSO. In this case, the solution without compound and kinase served as a blank control, corresponding to the "minimum value" shown below; the solution without compound but with kinase, adenosine 5'-triphosphate disodium salt hydrate, DMSO, and buffer served as a positive control, corresponding to the "maximum value" shown below.

4) Kinase reaction and termination: 10 μL of kinase buffer was added to a 384-well plate containing 5 μL of the compound to be tested, and the mixture was incubated at room temperature for 10 minutes. Another 10 μL of buffer containing the substrate peptide and adenosine 5'-triphosphate disodium salt hydrate was added to the 384-well plate. After incubation at 28°C for 1 hour, 25 μL of stop solution (100 mM HEPES pH 7.5, 50 mM EDTA, 0.2% Coating Reagent #3, and 0.015% Brij-35) was added to each well to terminate the reaction.

5) Data reading: CaliperEZ Reader II instrument was used to read the conversion rate data. Setting conditions: downstream voltage as -500 V, upstream voltage as -2250 V, base pressure as -0.5 PSI, and screening pressure as -1.2 PSI.

6) Data calculation: the conversion rate data was copied from CaliperEZ Reader II, and the conversion rate was converted into inhibition rate data. The calculation formula is as follows:

Inhibition percentage (%) = (maximum value - conversion rate) / (maximum value - minimum value) * 100%

IC50 values were fitted using XLFit excel add-in version 5.4.0.8.

Fitting formula: Y = Bottom + (Top - Bottom) / (1 + (IC50 / X) ^ HillSlope)

2.1.3 Experimental results

Kinase activity data for compound X are shown in Table 1.

Table 1: Kinase activity data for compound X

| No. | IRAK4 $IC_{50}$ (nM) |
|---|---|
| X | 26 |

**2.2 LPS-stimulated cytokine release in THP-1**

2.2.1 Experimental materials

| Product name | Supplier | Cat. No. |
|---|---|---|
| THP-1 | ATCC | TIB-202 |
| Dulbecco's Phosphate Buffered Saline (DPBS) | Biosera | LM-S2041/500 |
| RPMI 1640 medium | Thermo Fisher | 11875119 |
| Fetal bovine serum (FBS) | Biological Industries | 04-002-1A |
| Penicillin-Streptomycin Solution (P/S) | Invitrogen | 15140122 |
| β-Mercaptoethanol | Merck | M3148 |
| Dimethyl sulfoxide (DMSO) | Sigma | D2650 |
| Lipopolysaccharide (LPS) | Thermo Fisher | tlrl-pb5lps |
| 96-well cell culture plate | Corning | 3799 |
| Human TNF-α Duoset ELISA Kit | R&D | DY210 |

2.2.2 Detection of IRAK4 protein degradation in THP-1 induced by PROTAC

2.2.2.1 Based on the experimental method of immunoblot, *i.e.*, Western Blot, THP-1 cell samples processed by gel electrophoresis were stained with specific antibodies, and the degrading activity of the compound on the IRAK4 protein in THP-1 cells was determined by analyzing the position and depth of staining. The detection concentrations of the compound were 0 μM, 0.3 μM, 1 μM, and 3 μM, and the compound was used to treat the cells for 8 hours, 16 hours, 24 hours, and 48 hours. In this case, glyceraldehyde-3-phosphate dehydrogenase (GAPDH) was the internal reference protein for the assay.

2.2.2.2 Information on cells, reagents, and consumables is as follows:

| Name | Brand | Cat. No. |
|---|---|---|
| THP-1 cells | ATCC | TIB-202 |
| RPMI 1640 medium | Gibco | 22400-089 |
| Fetal bovine serum (FBS) | ExCell Bio | FSP500 |
| Penicillin-Streptomycin Solution (P/S) | HyClone | SV30010 |
| Pierce™ BCA Protein Assay Kit | Thermo Fisher | 23227 |
| RIPA lysis buffer | Merck | R0278 |
| Protease inhibitor (Complete Tablets EDTA-free, EASYpack) | Roche | 4693132001 |
| Phosphatase Inhibitor Cocktail 2 | Merck | P5726 |
| Phosphatase Inhibitor Cocktail 3 | Merck | P0044-5ML |
| Dimethyl sulfoxide (DMSO) | Merck | D2650 |
| NuPAGE™ MOPS SDS Running Buffer (20X) | Invitrogen | NP0001 |
| Pierce™ 20X TBS Tween™ 20 Buffer | Thermo Fisher | 28360 |
| NuPAGE™ 4 to 12% Bis-Tris, 1.5 mm, mini protein gel, 15-well | Invitrogen | NP0336BOX |

(continued)

| Name | Brand | Cat. No. |
|---|---|---|
| 1,4-Dithiothreitol (DTT) | Invitrogen | P2325 |
| Bovine serum albumin (BSA) | Merck | B2064-100G |
| Difco™ skim milk | BD Biosciences | 232100 |
| IBlot™ 2 Transfer Stack, nitrocellulose, regular size | Invitrogen | IB23001 |
| NuPAGE® LDS Sample Buffer (4×) | Invitrogen | NP0007 |
| PageRuler Prestained Protein Ladder | Thermo Fisher | 26616 |
| SuperSignal™ West Femto Maximum Sensitivity Substrate | Thermo Fisher | 34095 |
| Anti-IRAK4 antibody | Abcam | ab5985 |
| Anti-Glyceraldehyde-3-Phosphate Dehydrogenase Antibody, clone 6C5, GAPDH | Merck | MAB374 |
| Goat Anti-Rabbit IgG H&L (HRP) | Abcam | ab205718 |
| Goat Anti-Mouse IgG H&L (HRP) | Abcam | ab205719 |

2.2.2.3 Equipment

| Equipment name | Brand | Model |
|---|---|---|
| Cell counter | Count start | IC1000 |
| Multi-Mode Microplate Reader | Molecular Device | Molecular Device Flexstation III |
| Life technologies iBlot2 Gel Transfer Device | IB 21001 | 31252173 |
| Image Quant LAS 4000 | 399699 | Image Quant LAS 4010 |

2.2.2.4 Reagent preparation method

Running buffer: 50 mL of MOPS SDS Running Buffer (20X) and 50 mL of 20× TBS Tween-20 buffer were diluted to 1L with deionized water as running buffer;

5% skim milk (w/v): 5% skim milk was prepared by diluting 2.5 g of skim milk with 50 mL of 1× TBS Tween-20 buffer;

5% BSA (w/v): 5% BSA was prepared by diluting 2.5 g of BSA with 50 mL of 1× TBS Tween-20 buffer;

Anti-IRAK4 antibody was diluted at 1:1000 with 5% BSA into a primary antibody working solution;

Goat Anti-Rabbit IgG H&L (HRP) was diluted at 1:2000 with 5% BSA into a secondary antibody working solution;

Goat Anti-Mouse IgG H&L (HRP) was diluted at 1:2000 with 5% BSA into a secondary antibody working solution.

THP-1 cells were spread into a 6-well plate at a density of 1.5 × 106 cells per mL, and incubated in a 37°C cell culture incubator with 5% CO2 for 2 hours. The compound was diluted with DMSO to 0.6 mM, 0.2 mM, and 0.06 mM, respectively. 10 μL of compound solution was added to the corresponding wells, and the plate was incubated in the incubator for 16 hours, 24 hours, and 48 hours, respectively. The compound-treated THP-1 cells were collected from the wells, added with 120 μL of RIPA lysis buffer containing protease inhibitor, Phosphatase Inhibitor Cocktail 2, and Phosphatase Inhibitor Cocktail 3, and lysed on wet ice for 30 minutes. The cell lysate was then centrifuged at a low temperature and a high speed for 5 minutes to collect the supernatant. Referring to the instructions in the PierceTM BCA Protein Assay Kit, the cell samples were tested for protein concentration.

The samples were adjusted to the same concentration using lysis buffer and NuPAGE® LDS sample buffer

containing 1 M DTT. The samples were heated at 95°C for 5 minutes and centrifuged at a low temperature and a high speed. 20 μL of prepared protein sample and 4 μL of PageRuler Prestained Protein Ladder were added to the gel wells. The mixture was electrophoresed at a voltage of 80 V for 0.5 hours, and then electrophoresed for another 1.5 hours after the voltage was adjusted to 120 V. The gel was removed and proteins in the gel were transferred to IBlotTM 2 Transfer Stack at a voltage of 20V. After successful membrane transfer, the bands were cut out at 65 kDa to 40 kDa and 40 kDa to 30 kDa, respectively. The membrane was blocked with 5% skim milk at room temperature for 1 hour. The membrane was washed three times with 1X TBST, and incubated with IRAK4 antibody working solution at 4°C overnight. The IRAK4 antibody working solution was discarded, and the membrane was washed three times with 1X TBST. After the corresponding membrane was incubated with Goat Anti-Rabbit IgG H&L (HRP) working solution and Goat Anti-Mouse IgG H&L (HRP) working solution at room temperature for 1 hour, the antibody working solutions were discarded, and the membrane was washed three times with 1X TBST. Referring to the instructions in the SuperSignalTM West Femto Maximum Sensitivity Substrate Kit, the reagents in the kit were mixed in equal volume proportions to prepare a luminescent mixture, and the membrane was incubated for 1 minute and removed for exposure.

2.2.2.5 Experimental results

The Western Blot results 24 hours after administration of the compound at a concentration of 1 μM are shown in Table 2:

Table 2: IRAK4 degradation rate of compound X 24 hours after administration

| No. | IRAK4 degradation rate 24 hours after administration of the compound at a concentration of 1 μM |
|---|---|
| X | 75 to 100% |

The concentration that degraded 50% of IRAK4 by Western Blot 24 hours after administration is shown in Table 3:

Table 3: IRAK4 DC50 (nM) of compound X 24 hours after administration

| No. | IRAK4 $DC_{50}$ (nM) |
|---|---|
| X | 4.02 |

Example 3: Preparation and characterization of crystal form A

300 mg of compound X obtained in Example 1 was weighed into a 30 mL glass vial, and 5 mL of acetone was added thereto. The mixture was stirred at room temperature for 20 hours and filtered to obtain a yellow solid, which was dried in vacuum at 50°C for 4 hours. A total of 240 mg of crystal form A (yield: 80%) was obtained. The resulting solid was characterized by XRPD, DSC, and TGA. The results indicate that the resulting solid is crystal form A.

Table 4: List of XRPD peaks of crystal form A

| **No.** | **Angle (°)** | **d-value (Å)** | **Relative intensity** |
|---|---|---|---|
| 1 | 6.9 | 12.9 | 63.6% |
| 2 | 9.7 | 9.1 | 18.3% |
| 3 | 10.2 | 8.7 | 16.2% |
| 4 | 11.2 | 7.9 | 60.1% |
| 5 | 12.2 | 7.2 | 29.2% |
| 6 | 13.7 | 6.5 | 100.0% |
| 7 | 15.1 | 5.9 | 23.2% |
| 8 | 16.4 | 5.4 | 20.8% |
| 9 | 17.3 | 5.1 | 43.2% |
| 10 | 18.2 | 4.9 | 52.4% |
| 11 | 18.3 | 4.8 | 47.0% |
| 12 | 20.6 | 4.3 | 32.7% |

(continued)

| No. | Angle (°) | d-value (Å) | Relative intensity |
|---|---|---|---|
| 13 | 21.6 | 4.1 | 29.8% |
| 14 | 24.3 | 3.7 | 27.6% |
| 15 | 24.8 | 3.6 | 19.8% |

The XRPD of crystal form A is shown in Table 4 and FIG. 2. The DSC results indicate that crystal form A has an endothermic peak at 262.80°C, which is determined to be the melting point of crystal form A, as shown in FIG. 3. The TGA results indicate that crystal form A has a weight loss of 0.548% before 200°C, as shown in FIG. 4. The NMR results indicate no significant residual solvent.

**Example 4: Preparation and characterization of crystal form B**

30 mg of sample (compound X obtained in Example 1) was added to a 4 mL vial, followed by the addition of 0.5 mL of a solvent of DCM: MeOH (volume ratio of 3:1) and 1.1 equivalents of hydrochloric acid, and the mixture was stirred at room temperature for 24 hours. 1 mL of MTBE was added to precipitate a yellow solid, which was filtered and dried in vacuum at 50°C for 24 hours. The resulting solid was characterized by XRPD, DSC, and TGA. The results indicate that the resulting solid is crystal form B.

Table 5: List of XRPD peaks of crystal form B

| No. | Angle (°) | d-value (Å) | Relative intensity |
|---|---|---|---|
| 1 | 8.6 | 10.2 | 83.1% |
| 2 | 9.5 | 9.3 | 17.3% |
| 3 | 11.6 | 7.6 | 15.4% |
| 4 | 11.8 | 7.5 | 15.7% |
| 5 | 13.4 | 6.6 | 22.3% |
| 6 | 13.6 | 6.5 | 14.1% |
| 7 | 15.5 | 5.7 | 16.0% |
| 8 | 15.8 | 5.6 | 14.7% |
| 9 | 17.3 | 5.1 | 39.7% |
| 10 | 18.4 | 4.8 | 58.7% |
| 11 | 18.9 | 4.7 | 100.0% |
| 12 | 24.1 | 3.7 | 18.4% |
| 13 | 24.6 | 3.6 | 20.2% |
| 14 | 25.9 | 3.4 | 26.4% |
| 15 | 27.0 | 3.3 | 13.9% |
| 16 | 33.2 | 2.7 | 14.4% |

The XRPD of crystal form B is shown in Table 5 and FIG. 5. The DSC results indicate that crystal form B has endothermic peaks at 177.2°C and 243.4°C and an exothermic peak at 214.0°C. The TGA results indicate that crystal form B has two stages of weight loss, wherein the first stage shows a weight loss of 3.465% and the second stage shows a weight loss of 3.021%. The NMR results indicate no residual solvent or acid.

**Example 5: Preparation and characterization of crystal form C**

Approximately 100 mg of sample (compound X obtained in Example 1) was added to an 8 mL vial, followed by the addition of 2 mL of acetonitrile, and the mixture was stirred continuously at 50°C for 3 days to obtain a solid, which was filtered. The resulting solid was characterized by XRPD, DSC, and TGA. The results indicate that the resulting solid is

crystal form C.

Table 6: List of XRPD peaks of crystal form C

| No. | Angle (°) | d-value (Å) | Relative intensity |
|---|---|---|---|
| 1 | 9.7 | 9.1 | 34.5% |
| 2 | 10.8 | 8.2 | 25.5% |
| 3 | 12.5 | 7.1 | 29.3% |
| 4 | 13.1 | 6.8 | 35.7% |
| 5 | 14.1 | 6.3 | 48.6% |
| 6 | 14.7 | 6.0 | 12.3% |
| 7 | 15.2 | 5.8 | 22.6% |
| 8 | 15.5 | 5.7 | 33.4% |
| 9 | 16.8 | 5.3 | 21.6% |
| 10 | 18.7 | 4.7 | 100.0% |
| 11 | 19.6 | 4.5 | 55.4% |
| 12 | 19.8 | 4.5 | 57.1% |
| 13 | 20.7 | 4.3 | 69.5% |
| 14 | 21.7 | 4.1 | 27.0% |
| 15 | 22.4 | 4.0 | 45.7% |
| 16 | 24.3 | 3.7 | 13.1% |

The XRPD of crystal form C is shown in Table 6 and FIG. 6. The DSC results indicate that crystal form C has an endothermic peak at 261.8°C and an exothermic peak at 266.8°C, as shown in FIG. 7. The TGA results indicate that crystal form C has essentially no weight loss before 300°C, as shown in FIG. 8.

**Example 6: Preparation and characterization of crystal form D**

Experimental procedure: approximately 100 mg of sample (compound X obtained in Example 1) was added to an 8 mL vial, followed by the addition of 2 mL of 1,4-dioxane, and the mixture was stirred continuously at 50°C for 3 days to obtain a solid, which was filtered. The resulting solid was characterized by XRPD, DSC, and TGA. The results indicate that the resulting solid is crystal form D.

Table 7: List of XRPD peaks of crystal form D

| No. | Angle (°) | d-value (Å) | Relative intensity |
|---|---|---|---|
| 1 | 6.5 | 13.5 | 27.2% |
| 2 | 7.0 | 12.6 | 100.0% |
| 3 | 10.4 | 8.5 | 9.2% |
| 4 | 11.4 | 7.8 | 48.6% |
| 5 | 13.0 | 6.8 | 34.8% |
| 6 | 14.1 | 6.3 | 81.1% |
| 7 | 15.0 | 5.9 | 22.8% |
| 8 | 16.1 | 5.5 | 17.8% |
| 9 | 16.8 | 5.3 | 61.9% |
| 10 | 17.9 | 5.0 | 75.9% |
| 11 | 18.8 | 4.7 | 10.5% |

(continued)

| No. | Angle (°) | d-value (Å) | Relative intensity |
|---|---|---|---|
| 12 | 19.7 | 4.5 | 25.4% |
| 13 | 22.0 | 4.0 | 47.2% |
| 14 | 23.4 | 3.8 | 66.2% |
| 15 | 24.1 | 3.7 | 32.1% |
| 16 | 26.4 | 3.4 | 13.9% |

The XRPD of crystal form D is shown in Table 7 and FIG. 9. The DSC results indicate that crystal form D has endothermic peaks at 262.5°C and 284.2°C and an exothermic peak at 262.7°C. The TGA results indicate that crystal form D has essentially no weight loss before 300°C.

**Example 7: Preparation and characterization of crystal form F**

Approximately 100 mg of sample (compound X obtained in Example 1) was weighed into a 4 mL vial, and approximately 5 mL of DCM was added to a 30 mL sample vial. The open 4 mL vial containing the sample was placed in the 30 mL sample vial, which was sealed and allowed to stand at room temperature for 3 days to precipitate a solid. The resulting solid was characterized by XRPD, DSC, and TGA. The results indicate that the resulting solid is crystal form F.

Table 8: List of XRPD peaks of crystal form F

| No. | Angle (°) | d-value (Å) | Relative intensity |
|---|---|---|---|
| 1 | 5.8 | 15.3 | 20.4% |
| 2 | 6.6 | 13.4 | 29.1% |
| 3 | 7.2 | 12.2 | 60.6% |
| 4 | 9.8 | 9.0 | 8.9% |
| 5 | 10.1 | 8.7 | 6.2% |
| 6 | 11.2 | 7.9 | 15.6% |
| 7 | 12.2 | 7.2 | 56.5% |
| 8 | 13.3 | 6.7 | 100.0% |
| 9 | 13.6 | 6.5 | 37.6% |
| 10 | 14.5 | 6.1 | 28.2% |
| 11 | 15.7 | 5.7 | 74.0% |
| 12 | 16.2 | 5.5 | 30.9% |
| 13 | 16.6 | 5.3 | 13.9% |
| 14 | 17.5 | 5.1 | 43.5% |
| 15 | 18.3 | 4.8 | 17.5% |
| 16 | 18.9 | 4.7 | 39.1% |
| 17 | 20.0 | 4.4 | 24.5% |
| 18 | 20.8 | 4.3 | 38.9% |
| 19 | 21.7 | 4.1 | 17.6% |
| 20 | 22.5 | 3.9 | 48.3% |
| 21 | 24.1 | 3.7 | 18.1% |
| 22 | 24.6 | 3.6 | 21.4% |
| 23 | 26.5 | 3.4 | 9.4% |
| 24 | 27.0 | 3.3 | 10.4% |

(continued)

| No. | Angle (°) | d-value (Å) | Relative intensity |
|---|---|---|---|
| 25 | 28.9 | 3.1 | 3.4% |
| 26 | 35.5 | 2.5 | 3.3% |

The XRPD of crystal form F is shown in Table 8 and FIG. 10. The DSC results indicate that crystal form F has endothermic peaks at 249.1°C and 262.9°C and an exothermic peak at 258.6°C, as shown in FIG. 11. The TGA results indicate that crystal form F has essentially no weight loss before 300°C, as shown in FIG. 12.

**Example 8: Preparation and characterization of crystal form G**

Approximately 100 mg of sample (compound X obtained in Example 1) was weighed and dissolved in 0.8 mL of DMF, resulting in a saturated solution. *n*-Heptane was then added dropwise to the saturated solution until a solid was precipitated out. The resulting solid was characterized by XRPD, DSC, and TGA. The results indicate that the resulting solid is crystal form G.

Table 9: List of XRPD peaks of crystal form G

| No. | Angle (°) | d-value (Å) | Relative intensity |
|---|---|---|---|
| 1 | 6.6 | 13.3 | 12.1% |
| 2 | 9.0 | 9.9 | 66.3% |
| 3 | 13.4 | 6.6 | 46.8% |
| 4 | 13.9 | 6.4 | 5.6% |
| 5 | 14.2 | 6.2 | 7.3% |
| 6 | 14.5 | 6.1 | 4.9% |
| 7 | 15.7 | 5.6 | 9.8% |
| 8 | 16.9 | 5.2 | 4.9% |
| 9 | 17.2 | 5.2 | 5.9% |
| 10 | 18.1 | 4.9 | 27.2% |
| 11 | 19.3 | 4.6 | 100.0% |
| 12 | 20.2 | 4.4 | 6.4% |
| 13 | 20.6 | 4.3 | 17.7% |
| 14 | 21.2 | 4.2 | 8.0% |
| 15 | 24.8 | 3.6 | 16.2% |
| 16 | 26.2 | 3.4 | 4.6% |
| 17 | 26.7 | 3.3 | 2.0% |
| 18 | 27.3 | 3.3 | 22.0% |
| 19 | 28.2 | 3.2 | 10.4% |
| 20 | 30.1 | 3.0 | 6.9% |

The XRPD of crystal form G is shown in Table 9 and FIG. 13. The DSC results indicate that crystal form G has endothermic peaks at 124.9°C, 259.5°C, and 289.3°C, and an exothermic peak at 264.4°C. The TGA results indicate that crystal form G has a weight loss of 7.95%.

**Example 9: Preparation and characterization of crystal form H**

Approximately 100 mg of sample (compound X obtained in Example 1) was weighed and dissolved in 0.8 mL of DMF at room temperature, resulting in a saturated solution. Toluene was then added dropwise to the saturated solution

until a solid was precipitated out, wherein the volume ratio of toluene to DMF was approximately 10:1. The resulting solid was characterized by XRPD, DSC, and TGA. The results indicate that the resulting solid is crystal form H.

Table 10: List of XRPD peaks of crystal form H

| No. | Angle (°) | d-value (Å) | Relative intensity |
|---|---|---|---|
| 1 | 5.8 | 15.3 | 5.7% |
| 2 | 8.5 | 10.4 | 18.7% |
| 3 | 9.5 | 9.3 | 9.6% |
| 4 | 10.9 | 8.1 | 37.0% |
| 5 | 11.7 | 7.6 | 3.7% |
| 6 | 14.1 | 6.3 | 12.0% |
| 7 | 14.4 | 6.1 | 3.7% |
| 8 | 14.9 | 6.0 | 2.6% |
| 9 | 15.5 | 5.7 | 8.2% |
| 10 | 15.9 | 5.6 | 9.7% |
| 11 | 16.6 | 5.3 | 7.9% |
| 12 | 16.9 | 5.2 | 85.1% |
| 13 | 17.2 | 5.2 | 32.5% |
| 14 | 17.6 | 5.0 | 15.6% |
| 15 | 18.5 | 4.8 | 17.0% |
| 16 | 19.1 | 4.6 | 28.2% |
| 17 | 20.8 | 4.3 | 100.0% |
| 18 | 21.8 | 4.1 | 8.1% |
| 19 | 22.5 | 3.9 | 3.4% |
| 20 | 23.0 | 3.9 | 6.1% |
| 21 | 23.3 | 3.8 | 4.8% |
| 22 | 23.9 | 3.7 | 6.7% |
| 23 | 24.5 | 3.6 | 6.0% |
| 24 | 25.4 | 3.5 | 7.5% |
| 25 | 25.8 | 3.5 | 42.5% |
| 26 | 26.5 | 3.4 | 11.0% |
| 27 | 27.4 | 3.3 | 3.6% |
| 28 | 28.0 | 3.2 | 3.9% |
| 29 | 28.2 | 3.2 | 4.8% |
| 30 | 32.4 | 2.8 | 3.1% |
| 31 | 33.0 | 2.7 | 3.3% |

The XRPD of crystal form H is shown in Table 10 and FIG. 14. The DSC results indicate that crystal form H has an endothermic peak at 288.5°C, as shown in FIG. 15. The TGA results indicate that crystal form H has essentially no weight loss before 300°C, as shown in FIG. 16.

**Example 10: Preparation and characterization of crystal form I**

Approximately 100 mg of sample (compound X obtained in Example 1) was weighed and dissolved in 0.8 mL of

DMSO at room temperature, resulting in a saturated solution. MTBE was then added dropwise to the saturated solution until a solid was precipitated out. The resulting solid was characterized by XRPD, DSC, and TGA. The results indicate that the resulting solid is crystal form I.

Table 11: List of XRPD peaks of crystal form I

| No. | Angle (°) | d-value (Å) | Relative intensity |
|---|---|---|---|
| 1 | 7.6 | 11.6 | 11.0% |
| 2 | 8.0 | 11.1 | 41.7% |
| 3 | 11.3 | 7.8 | 100.0% |
| 4 | 12.1 | 7.3 | 81.3% |
| 5 | 15.5 | 5.7 | 21.0% |
| 6 | 16.0 | 5.5 | 31.3% |
| 7 | 16.5 | 5.4 | 28.7% |
| 8 | 17.0 | 5.2 | 13.0% |
| 9 | 17.6 | 5.0 | 47.4% |
| 10 | 18.6 | 4.8 | 18.4% |
| 11 | 20.2 | 4.4 | 51.3 |
| 12 | 21.1 | 4.2 | 11.3% |
| 13 | 21.5 | 4.1 | 10.9% |
| 14 | 22.0 | 4.0 | 49.1% |
| 15 | 22.8 | 3.9 | 17.7% |
| 16 | 23.2 | 3.8 | 11.2% |
| 17 | 24.2 | 3.7 | 15.3% |
| 18 | 25.3 | 3.5 | 49.8% |
| 19 | 25.6 | 3.5 | 21.0% |
| 20 | 26.8 | 3.3 | 12.0% |
| 21 | 28.5 | 3.1 | 12.6% |
| 22 | 31.7 | 2.8 | 4.9% |

The XRPD of crystal form I is shown in Table 11 and FIG. 17. The DSC results indicate that crystal form I has an endothermic peak at 279.6°C, corresponding to the melting point, as shown in FIG. 18. The TGA results indicate that crystal form I has a weight loss of 0.865% before 150°C, as shown in FIG. 19.

**Example 11: Preparation and characterization of crystal form J**

Approximately 100 mg of sample (compound X obtained in Example 1) was dissolved in 1 mL of DMSO at 50°C, then 0.3 mL of a mixed solvent of EtOH: water (volume ratio of 4:1) was added dropwise thereto, and the mixture was kept stable for 2 hours to precipitate a solid, which was filtered. The resulting solid was characterized by XRPD, DSC, and TGA. The results indicate that the resulting solid is crystal form J.

Table 12: List of XRPD peaks of crystal form J

| No. | Angle (°) | d-value (Å) | Relative intensity |
|---|---|---|---|
| 1 | 6.4 | 13.7 | 50.3% |
| 2 | 9.6 | 9.2 | 18.9% |
| 3 | 12.9 | 6.8 | 30.5% |
| 4 | 14.5 | 6.1 | 9.1% |

(continued)

| No. | Angle (°) | d-value (Å) | Relative intensity |
|---|---|---|---|
| 5 | 14.8 | 6.0 | 13.7% |
| 6 | 15.5 | 5.7 | 7.8% |
| 7 | 16.5 | 5.4 | 11.6% |
| 8 | 16.9 | 5.2 | 12.0% |
| 9. | 17.7 | 5.0 | 26.4% |
| 10 | 18.2 | 4.9 | 20.5% |
| 11 | 18.7 | 4.7 | 100.0% |
| 12 | 19.0 | 4.7 | 94.6% |
| 13 | 19.2 | 4.6 | 82.5% |
| 14 | 19.9 | 4.5 | 11.5% |
| 15 | 20.3 | 4.4 | 11.0% |
| 16 | 20.9 | 4.3 | 57.0% |
| 17 | 21.3 | 4.2 | 36.8% |
| 18 | 22.4 | 4.0 | 24.3% |
| 19 | 22.8 | 3.9 | 55.0% |
| 20 | 23.3 | 3.8 | 32.5% |
| 21 | 23.9 | 3.7 | 30.4% |
| 22 | 24.1 | 3.7 | 32.5% |
| 23 | 25.2 | 3.5 | 42.1% |
| 24 | 25.9 | 3.4 | 45.9% |
| 25 | 27.5 | 3.2 | 8.8% |
| 26 | 28.8 | 3.1 | 28.2% |
| 27 | 29.6 | 3.0 | 12.1% |
| 28 | 30.2 | 3.0 | 10.4% |
| 29 | 31.9 | 2.8 | 7.3% |
| 30 | 35.1 | 2.6 | 6.2% |
| 31 | 36.8 | 2.4 | 6.3% |

The XRPD of crystal form J is shown in Table 12 and FIG. 20. The DSC results indicate that crystal form J has endothermic peaks at 181.1 °C, 216.1°C, 264.5°C, and 290.4°C, and exothermic peaks at 223.8°C and 267.4°C. The TGA results indicate that crystal form J has a weight loss of 1.726% before 220°C.

**Example 12: Effects of crystal form A and crystal form I**

12.1 Liquid phase method

The solubility of crystal form A and crystal form I and the stability of crystal form I were tested using an Agilent 1200 HPLC (Agilent, USA) equipped with a DAD detector for solubility analysis on samples. Detailed chromatographic conditions are as follows:

| **Chromatographic column** | CAPCELL ADME-HR, 4.6 * 250 mm, 5 μm |
|---|---|
| **Flow rate** | 0.8 mL/min |
| **Column temperature** | 30°C |

(continued)

| **Injection volume** | 5 μL | | |
|---|---|---|---|
| **Mobile phase** | A: 0.05% phosphoric acid aqueous solution | | |
| | B: 0.05% phosphoric acid/acetonitrile solution | | |
| **Gradient** | Time (min) | A (%) | B (%) |
| | 0.00 | 85.0 | 15.0 |
| | 5.00 | 85.0 | 15.0 |
| | 10.00 | 60.0 | 40.0 |
| | 19.00 | 35.0 | 65.0 |
| | 21.00 | 20.0 | 80.0 |
| | 33.00 | 10.0 | 90.0 |
| | 38.00 | 10.0 | 90.0 |
| | 38.10 | 85.0 | 15.0 |
| | 45.00 | 85.0 | 15.0 |
| **Run time** | 45 min | | |
| **Detection wavelength** | 214 nm | | |
| **Diluent** | ACN: water = 9:1 (volume ratio) | | |

12.2 Solubility of crystal form A and crystal form I

The solubility of crystal form A and crystal form I was evaluated by preparing 5 mg/mL suspensions of the two crystal forms in pH 1.2 buffer, pH 3.0 buffer, pH 4.5 buffer, pH 6.8 buffer, pH 7.4 buffer, simulated gastric fluid (SGF), fasted state simulated intestinal fluid (FaSSIF), fed state simulated intestinal fluid (FeSSIF), and water, respectively. The suspensions were stirred at 37°C for 24 hours. The solubility of the two crystal forms at 2 hours and 24 hours and the pH of the solution at 24 hours were tested. The solid was filtered, and the resulting solid was tested for XRPD. The results are shown in Table 13.

Table 13: Evaluation of solubility of crystal form A and crystal form I

| **Crystal form** | **Medium** | **Original pH** | **pH (24 h)** | **Solubility at 37°C (mg/mL)** | | **Crystal form** |
|---|---|---|---|---|---|---|
| | | | | **2 h** | **24 h** | |
| Crystal form A | pH 1.2 | 1.214 | 1.214 | <LOQ | <LOQ | Crystal form A |
| | pH 3.0 | 3.024 | 3.078 | <LOQ | <LOQ | Crystal form A |
| | pH 4.5 | 4.545 | 4.584 | 0.00026 | <LOQ | Crystal form A |
| | pH 6.8 | 6.831 | 6.833 | <LOQ | <LOQ | Crystal form A |
| | pH 7.4 | 7.429 | 7.428 | <LOQ | <LOQ | Crystal form A |
| | FaSSIF | 6.549 | 6.570 | 0.00461 | 0.00285 | Crystal form A |
| | FeSSIF | 5.003 | 5.032 | 0.00391 | 0.00414 | Crystal form A |
| | SGF | 1.213 | 1.225 | <LOQ | <LOQ | Crystal form A |
| | Water | 7.010 | 7.311 | 0.00052 | 0.00075 | Crystal form A |

(continued)

| Crystal form | Medium | Original pH | pH (24 h) | Solubility at 37°C (mg/mL) | | Crystal form |
|---|---|---|---|---|---|---|
| | | | | 2 h | 24 h | |
| Crystal form I | pH 1.2 | 1.205 | 1.234 | 0.00084 | 0.00081 | Crystal form I |
| | pH 3.0 | 3.046 | 3.145 | 0.00048 | 0.00077 | Crystal form I |
| | pH 4.5 | 4.500 | 4.583 | 0.00019 | 0.00087 | Crystal form I |
| | pH 6.8 | 6.796 | 6.805 | 0.00046 | 0.00084 | Crystal form I |
| | pH 7.4 | 7.438 | 7.439 | 0.00101 | 0.00079 | Crystal form I |
| | FaSSIF | 6.469 | 6.501 | 0.00141 | 0.00148 | Crystal form I |
| | FeSSIF | 5.032 | 5.085 | 0.00400 | 0.01099 | Crystal form I |
| | SGF | 1.245 | 1.266 | 0.00158 | 0.00376 | Crystal form I |
| | Water | 6.578 | 6.749 | 0.00089 | 0.00025 | Crystal form I |
| Note: LOQ refers to the limit of quantitation. | | | | | | |

It can be seen from the results that crystal form A can achieve a solubility of approximately 0.004 mg/mL in FaSSIF and FeSSIF, and crystal form I can achieve a solubility of approximately 0.01 mg/mL in FeSSIF under stirring at 37°C for 24 hours. Both crystal form A and crystal form I remained unchanged in each medium.

12.3 Evaluation of physicochemical stability of crystal form A

12.3.1 Liquid phase method

Samples were tested for their solubility using an Agilent 1200 HPLC (Agilent, USA) equipped with a DAD detector. Detailed chromatographic conditions are as follows:

| **Chromatographic column** | CAPCELL ADME-HR, 4.6 * 250 mm, 5 μm | | |
|---|---|---|---|
| **Flow rate** | 1.0 mL/min | | |
| **Column temperature** | 30°C | | |
| **Injection volume** | 5 μL | | |
| **Mobile phase** | A: 0.1% TFA aqueous solution | | |
| | B: 0.1% TFA/acetonitrile solution | | |
| **Gradient** | Time (min) | A (%) | B (%) |
| | 0.00 | 70.0 | 30.0 |
| | 5.00 | 70.0 | 30.0 |
| | 7.00 | 60.0 | 40.0 |
| | 17.00 | 20.0 | 80.0 |
| | 33.00 | 5.0 | 95.0 |
| | 38.00 | 5.0 | 95.0 |
| | 38.10 | 70.0 | 30.0 |
| | 45.00 | 70.0 | 30.0 |
| **Run time** | 45 min | | |
| **Detection wavelength** | 266 nm | | |
| **Diluent** | ACN: water = 9:1 (volume ratio) | | |

12.3.2 Stability testing

Samples of crystal form A (obtained in Example 3) were weighed into a liquid phase vial to prepare 5 parts. These samples were placed in 60°C (closed), 25°C/60% RH (open), 40°C/75% RH (open), and light stability (closed) chambers, respectively. The physicochemical stability of the samples was measured after 10 days under light exposure and 1 week under 60°C, 25°C/60% RH, and 40°C/75% RH conditions. The results are shown in Table 14.

Table 14: Evaluation of stability of crystal form A

| Condition | Initial | 25°C/60% RH, open | 40°C/75% RH, open | 60°C, closed | Light, closed | Light, control |
|---|---|---|---|---|---|---|
| Time | N/A | 1 week | 1 week | 1 week | 10 days | 10 days |
| Purity (%) | 96.41 | 96.38 | 96.39 | 96.40 | 96.41 | 96.42 |
| XRPD | Crystal form A | Unchanged | Unchanged | Unchanged | Unchanged | Unchanged |
| Note: N/A means not applicable. | | | | | | |

It can be seen from the results that crystal form A exhibited no significant degradation under all stability conditions, with no change in the crystal form, indicating good physicochemical stability.

12.4 Evaluation of physicochemical stability of crystal form I

Approximately 10 mg of samples of crystal form I (obtained in Example 10) were weighed into a liquid phase vial to prepare 5 parts. These samples were placed in 60°C (closed), 25°C/60% RH (open), 40°C/75% RH (open), and light (closed) stability chambers, respectively. The physicochemical stability of the samples was measured after 10 days under light exposure and 1 week under 60°C, 25°C/60% RH, and 40°C/75% RH conditions. The results are shown in Table 15.

Table 15: Evaluation of stability of crystal form I

| Condition | Time | Purity (%) | XRPD |
|---|---|---|---|
| Initial | N/A | 98.97 | N/A |
| 25°C/60% RH | 1 week | 98.96 | Unchanged |
| 40°C/75% RH | 1 week | 98.96 | Unchanged |
| 60°C | 1 week | 98.80 | Unchanged |
| Light | 10 days | 98.96 | Unchanged |
| Light, control | 10 days | 98.97 | Unchanged |
| Note: N/A means not applicable. | | | |

It can be seen from the results that crystal form I showed a 0.17% degradation in the closed stability chamber at 60°C for 1 week and exhibited no significant degradation under other stability conditions, with no change in the crystal form, indicating good physicochemical stability.

12.5 Evaluation of mechanical stability of crystal form A and crystal form I

The starting samples, *i.e.*, crystal form A (obtained in Example 3) and crystal form I (obtained in Example 10), were subjected to grinding and tableting experiments, and the resulting solids were subjected to XRPD testing. The results are shown in Table 16.

Dry grinding: approximately 10 mg of the starting sample was ground for 3 minutes in a mortar, and then subjected to XRPD testing.

Wet grinding: approximately 10 mg of the starting sample was ground with 1 to 2 drops of solvent for 3 minutes in a mortar, and then subjected to XRPD testing.

Tableting experiment: approximately 10 mg of the starting sample was compressed at 20 MPa for 3 minutes, and then subjected to XRPD testing.

Table 16: Summary of mechanical stability experiments of crystal form A and crystal form I

| Starting material | Processing method | Solvent | Solid color | XRPD results |
|---|---|---|---|---|
| Crystal form A | Dry grinding | N/A | Slightly deepened, yellow | Crystal form A (low degree of crystallinity) |
| Crystal form I | | | Yellow, essentially no change | Crystal form I (low degree of crystallinity) |
| Crystal form A | Wet grinding | EtOH | Slightly deepened, yellow | Crystal form A |
| Crystal form I | | | Yellow, essentially no change | Crystal form I |
| Crystal form A | | Water | Slightly deepened, yellow | Crystal form A |
| Crystal form I | | | Yellow, essentially no change | Crystal form I |
| Crystal form A | Tableting | EA | Slightly deepened, yellow | Crystal form A |
| Crystal form I | | | Yellow, essentially no change | Crystal form I |

It can be seen from the results that crystal form A and crystal form I showed no change in the crystal form of the solid obtained after dry grinding, wet grinding, and tableting, but exhibited a reduction in the degree of crystallinity of the solid obtained after dry grinding, and almost no change in the degree of crystallinity of the solid obtained after wet grinding and tableting. The solid color of crystal form A after grinding and tableting experiments was slightly darker than the yellow color exhibited by the starting sample, while there was essentially no change in the solid color of crystal form I.

12.6 Evaluation of hygroscopicity of crystal form I

Crystal form I (obtained in Example 10) was subjected to DVS testing. The results are shown in FIG. 21 and FIG. 22.

It can be seen from the results that crystal form I exhibited a hygroscopic weight gain of 0.3629% at 80% RH, which was evaluated as slightly hygroscopic, and showed no change in the crystal form after DVS testing.

12.7 Evaluation of hygroscopicity of crystal form A

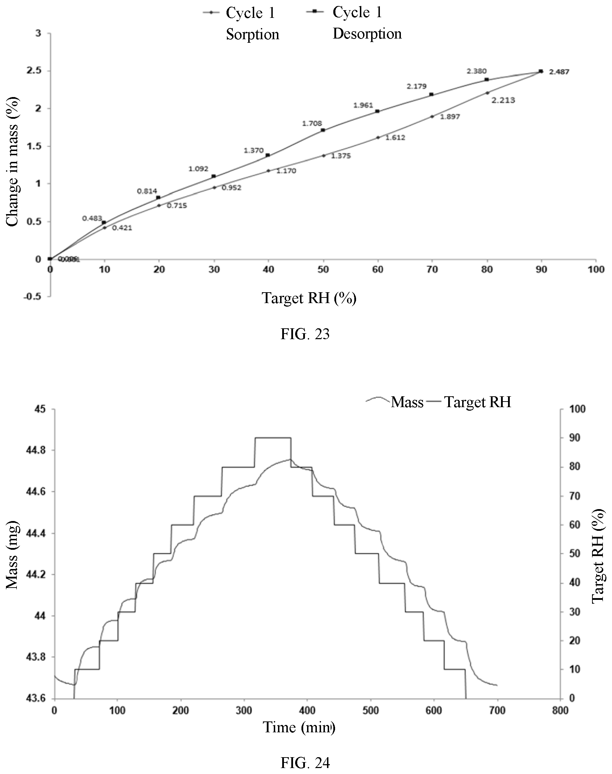

Crystal form A (obtained in Example 3) was subjected to DVS testing. The results are shown in FIG. 23 and FIG. 24.

It can be seen from the results that crystal form A exhibited a hygroscopic weight gain of 2.213% at 80% RH, which was evaluated as hygroscopic, and showed no change in the crystal form after DVS testing.

**Example 13: Hydrochloride of compound X**

13.1 Preparation

300 mg of compound X (obtained in Example 1) was weighed into a 30 mL glass vial, and 9 mL of ethyl acetate was added thereto. 1.1 equivalents of hydrochloric acid was weighed and dissolved in 1 mL of ethyl acetate, and the mixture was slowly added dropwise to the suspension. The mixture was stirred at room temperature for 48 hours and filtered to obtain a yellow solid, which was dried in vacuum at 50°C for 24 hours. A total of 270 mg of hydrochloride of compound X (yield: 86%) was obtained.

13.2 Identification

The hydrochloride obtained in 13.1 was tested by ion chromatography. The results showed a chloride ion content of 3.3586% and a ratio of hydrochloric acid to free base of approximately 0.8:1 (acid: free base).

**Example 14: Sulfate of compound X**

14.1 Preparation

300 mg of compound X (obtained in Example 1) was weighed into a 30 mL glass vial, and 4 mL of a mixed solvent of DCM: MeOH (volume ratio of 3:1) was added until dissolved. 1.1 equivalents of sulfuric acid was weighed into 1 mL of the above mixed solvent, mixed well, and slowly added dropwise to the mixture. The mixture was stirred at room temperature for 40 hours, and the solution became clear. 5 mL of MTBE was slowly added dropwise thereto, and the solution became turbid. The mixture was stirred at room temperature for another 8 hours and filtered to obtain a yellow solid, which was dried in vacuum at 50°C for 24 hours. A total of 250 mg of sulfate of compound X (yield: 75%) was obtained.

14.2 Identification

The sulfate obtained in 14.1 was tested by ion chromatography. The results showed a sulfate ion content of 4.8766% and a salt formation ratio of approximately 0.5:1 (acid: free base).

## Claims

1. A crystal form A, crystal form B, crystal form C, crystal form D, crystal form F, crystal form G, crystal form H, crystal form I, or crystal form J of a five-membered-fused six-membered compound of formula X, wherein

X

the crystal form A has an X-ray powder diffraction pattern using Cu-Kα radiation and represented by 2θ angles comprising diffraction peaks at 6.9 ± 0.2°, 11.2 ± 0.2°, 12.2 ± 0.2°, 13.7 ± 0.2°, 17.3 ± 0.2°, 18.2 ± 0.2°, and 24.3 ± 0.2°;
the crystal form C has an X-ray powder diffraction pattern using Cu-Kα radiation and represented by 2θ angles comprising diffraction peaks at 9.7 ± 0.2°, 10.8 ± 0.2°, 13.1 ± 0.2°, 14.1 ± 0.2°, 18.7 ± 0.2°, and 21.7 ± 0.2°;
the crystal form F has an X-ray powder diffraction pattern using Cu-Kα radiation and represented by 2θ angles comprising diffraction peaks at 5.8 ± 0.2°, 6.6 ± 0.2°, 7.2 ± 0.2°, 12.2 ± 0.2°, 14.5 ± 0.2°, 15.7 ± 0.2°, 18.9 ± 0.2°, and 24.6 ± 0.2°;
the crystal form H has an X-ray powder diffraction pattern using Cu-Kα radiation and represented by 2θ angles comprising diffraction peaks at 5.8 ± 0.2°, 8.5 ± 0.2°, 9.5 ± 0.2°, 11.7 ± 0.2°, 14.1 ± 0.2°, 16.9 ± 0.2°, and 20.8 ± 0.2°;
the crystal form I has an X-ray powder diffraction pattern using Cu-Kα radiation and represented by 2θ angles comprising diffraction peaks at 8.0 ± 0.2°, 11.3 ± 0.2°, 15.5 ± 0.2°, 17.6 ± 0.2°, 20.2 ± 0.2°, and 25.6 ± 0.2°;
the crystal form B has an X-ray powder diffraction pattern using Cu-Kα radiation and represented by 2θ angles comprising diffraction peaks at 8.6 ± 0.2°, 11.8 ± 0.2°, 15.8 ± 0.2°, and 18.9 ± 0.2°;
the crystal form D has an X-ray powder diffraction pattern using Cu-Kα radiation and represented by 2θ angles comprising diffraction peaks at 6.5 ± 0.2°, 7.0 ± 0.2°, 11.4 ± 0.2°, 13.0 ± 0.2°, 17.9 ± 0.2°, and 22.0 ± 0.2°;
the crystal form G has an X-ray powder diffraction pattern using Cu-Kα radiation and represented by 2θ angles comprising diffraction peaks at 9.0 ± 0.2°, 13.4 ± 0.2°, 14.5 ± 0.2°, 19.3 ± 0.2°, and 24.8 ± 0.2°;
the crystal form J has an X-ray powder diffraction pattern using Cu-Kα radiation and represented by 2θ angles comprising diffraction peaks at 6.4 ± 0.2°, 9.6 ± 0.2°, 12.9 ± 0.2°, 16.5 ± 0.2°, 17.7 ± 0.2°, and 22.8 ± 0.2°.

2. The crystal form A, crystal form B, crystal form C, crystal form D, crystal form F, crystal form G, crystal form H, crystal form I, or crystal form J according to claim 1, which satisfies one or more of the following conditions:

(1) the X-ray powder diffraction pattern for the crystal form A, using Cu-Kα radiation and represented by 2θ angles, further comprises diffraction peaks at one or more of 9.7 ± 0.2°, 10.2 ± 0.2°, 15.1 ± 0.2°, and 20.6 ± 0.2°;

(2) the X-ray powder diffraction pattern for the crystal form C, using Cu-K$\alpha$ radiation and represented by 2$\theta$ angles, further comprises diffraction peaks at one or more of 12.5 ± 0.2°, 15.5 ± 0.2°, and 24.3 ± 0.2°;
(3) the X-ray powder diffraction pattern for the crystal form F, using Cu-K$\alpha$ radiation and represented by 2$\theta$ angles, further comprises diffraction peaks at one or more of 9.8 ± 0.2°, 13.3 ± 0.2°, 17.5 ± 0.2°, and 20.8 ± 0.2°;
(4) the X-ray powder diffraction pattern for the crystal form H, using Cu-K$\alpha$ radiation and represented by 2$\theta$ angles, further comprises diffraction peaks at one or more of 10.9 ± 0.2°, 19.1 ± 0.2°, 26.5 ± 0.2°, and 33.0 ± 0.2°;
(5) the X-ray powder diffraction pattern for the crystal form I, using Cu-K$\alpha$ radiation and represented by 2$\theta$ angles, further comprises diffraction peaks at one or more of 7.6 ± 0.2°, 18.6 ± 0.2°, 22.0 ± 0.2°, and 31.7 ± 0.2°;
(6) the X-ray powder diffraction pattern for the crystal form B, using Cu-K$\alpha$ radiation and represented by 2$\theta$ angles, further comprises diffraction peaks at 17.3 ± 0.2° and/or 25.9 ± 0.2°;
(7) the X-ray powder diffraction pattern for the crystal form D, using Cu-K$\alpha$ radiation and represented by 2$\theta$ angles, further comprises diffraction peaks at one or more of 10.4 ± 0.2°, 16.8 ± 0.2°, and 26.4 ± 0.2°;
(8) the X-ray powder diffraction pattern for the crystal form G, using Cu-K$\alpha$ radiation and represented by 2$\theta$ angles, further comprises diffraction peaks at one or more of 16.9 ± 0.2°, 20.6 ± 0.2°, and 27.3 ± 0.2°;
(9) the X-ray powder diffraction pattern for the crystal form J, using Cu-K$\alpha$ radiation and represented by 2$\theta$ angles, further comprises diffraction peaks at one or more of 16.9 ± 0.2°, 20.9 ± 0.2°, 25.9 ± 0.2°, and 28.8 ± 0.2°;
(10) the crystal form A has a differential scanning calorimetry pattern comprising an endothermic peak at 262.80 ± 5°C;
(11) the crystal form A has a thermogravimetric analysis pattern with a weight loss of 0.548% before 200 ± 5°C;
(12) the crystal form C has a differential scanning calorimetry pattern comprising an endothermic peak at 261.8 ± 5°C and an exothermic peak at 266.8 ± 5°C;
(13) the crystal form C has a thermogravimetric analysis pattern with essentially no weight loss before 300 ± 5°C;
(14) the crystal form F has a differential scanning calorimetry pattern comprising endothermic peaks at 249.1 ± 5°C and 262.9 ± 5°C and an exothermic peak at 258.6 ± 5°C;
(15) the crystal form F has a thermogravimetric analysis pattern with essentially no weight loss before 300 ± 5°C;
(16) the crystal form H has a differential scanning calorimetry pattern comprising an endothermic peak at 288.5 ± 5°C;
(17) the crystal form H has a thermogravimetric analysis pattern with essentially no weight loss before 300 ± 5°C;
(18) the crystal form I has a differential scanning calorimetry pattern comprising an endothermic peak at 279.6 ± 5°C; and
(19) the crystal form I has a thermogravimetric analysis pattern with a weight loss of 0.865% before 150 ± 5°C.

**3.** The crystal form A, crystal form B, crystal form C, crystal form D, crystal form F, crystal form G, crystal form H, crystal form I, or crystal form J according to claim 2, which satisfies one or more of the following conditions:

(1) the X-ray powder diffraction pattern for the crystal form A, using Cu-K$\alpha$ radiation and represented by 2$\theta$ angles, has positions of diffraction peaks as shown in the following table:

| No. | Angle (°) | No. | Angle (°) |
|---|---|---|---|
| 1 | 6.9 | 9 | 17.3 |
| 2 | 9.7 | 10 | 18.2 |
| 3 | 10.2 | 11 | 18.3 |
| 4 | 11.2 | 12 | 20.6 |
| 5 | 12.2 | 13 | 21.6 |
| 6 | 13.7 | 14 | 24.3 |
| 7 | 15.1 | 15 | 24.8 |
| 8 | 16.4 | | |

(2) the X-ray powder diffraction pattern for the crystal form C, using Cu-K$\alpha$ radiation and represented by 2$\theta$ angles, has positions of diffraction peaks as shown in the following table:

| No. | Angle (°) | No. | Angle (°) |
|---|---|---|---|
| 1 | 9.7 | 9 | 16.8 |

(continued)

| No. | Angle (°) | No. | Angle (°) |
|---|---|---|---|
| 2 | 10.8 | 10 | 18.7 |
| 3 | 12.5 | 11 | 19.6 |
| 4 | 13.1 | 12 | 19.8 |
| 5 | 14.1 | 13 | 20.7 |
| 6 | 14.7 | 14 | 21.7 |
| 7 | 15.2 | 15 | 22.4 |
| 8 | 15.5 | 16 | 24.3 |

;

(3) the X-ray powder diffraction pattern for the crystal form F, using Cu-Kα radiation and represented by 2θ angles, has positions of diffraction peaks as shown in the following table:

| No. | Angle (°) | No. | Angle (°) |
|---|---|---|---|
| 1 | 5.8 | 14 | 17.5 |
| 2 | 6.6 | 15 | 18.3 |
| 3 | 7.2 | 16 | 18.9 |
| 4 | 9.8 | 17 | 20.0 |
| 5 | 10.1 | 18 | 20.8 |
| 6 | 11.2 | 19 | 21.7 |
| 7 | 12.2 | 20 | 22.5 |
| 8 | 13.3 | 21 | 24.1 |
| 9 | 13.6 | 22 | 24.6 |
| 10 | 14.5 | 23 | 26.5 |
| 11 | 15.7 | 24 | 27.0 |
| 12 | 16.2 | 25 | 28.9 |
| 13 | 16.6 | 26 | 35.5 |

;

(4) the X-ray powder diffraction pattern for the crystal form H, using Cu-Kα radiation and represented by 2θ angles, has positions of diffraction peaks as shown in the following table:

| No. | Angle (°) | No. | Angle (°) |
|---|---|---|---|
| 1 | 5.8 | 17 | 20.8 |
| 2 | 8.5 | 18 | 21.8 |
| 3 | 9.5 | 19 | 22.5 |
| 4 | 10.9 | 20 | 23.0 |
| 5 | 11.7 | 21 | 23.3 |
| 6 | 14.1 | 22 | 23.9 |
| 7 | 14.4 | 23 | 24.5 |
| 8 | 14.9 | 24 | 25.4 |
| 9 | 15.5 | 25 | 25.8 |

(continued)

| No. | Angle (°) | No. | Angle (°) |
|---|---|---|---|
| 10 | 15.9 | 26 | 26.5 |
| 11 | 16.6 | 27 | 27.4 |
| 12 | 16.9 | 28 | 28.0 |
| 13 | 17.2 | 29 | 28.2 |
| 14 | 17.6 | 30 | 32.4 |
| 15 | 18.5 | 31 | 33.0 |
| 16 | 19.1 | | |

(5) the X-ray powder diffraction pattern for the crystal form I, using Cu-K$\alpha$ radiation and represented by 2$\theta$ angles, has positions of diffraction peaks as shown in the following table:

| No. | Angle (°) | No. | Angle (°) |
|---|---|---|---|
| 1 | 7.6 | 12 | 21.1 |
| 2 | 8.0 | 13 | 21.5 |
| 3 | 11.3 | 14 | 22.0 |
| 4 | 12.1 | 15 | 22.8 |
| 5 | 15.5 | 16 | 23.2 |
| 6 | 16.0 | 17 | 24.2 |
| 7 | 16.5 | 18 | 25.3 |

(6) the X-ray powder diffraction pattern for the crystal form B, using Cu-K$\alpha$ radiation and represented by 2$\theta$ angles, has positions and relative intensities of diffraction peaks as shown in the following table:

| No. | Angle (°) | Relative intensity | No. | Angle (°) | Relative intensity |
|---|---|---|---|---|---|
| 1 | 8.6 | 83.1% | 9 | 17.3 | 39.7% |
| 2 | 9.5 | 17.3% | 10 | 18.4 | 58.7% |
| 3 | 11.6 | 15.4% | 11 | 18.9 | 100.0% |
| 4 | 11.8 | 15.7% | 12 | 24.1 | 18.4% |
| 5 | 13.4 | 22.3% | 13 | 24.6 | 20.2% |
| 6 | 13.6 | 14.1% | 14 | 25.9 | 26.4% |
| 7 | 15.5 | 16.0% | 15 | 27.0 | 13.9% |
| 8 | 15.8 | 14.7% | 16 | 33.2 | 14.4% |

(7) the X-ray powder diffraction pattern for the crystal form D, using Cu-K$\alpha$ radiation and represented by 2$\theta$ angles, has positions and relative intensities of diffraction peaks as shown in the following table:

| No. | Angle (°) | Relative intensity | No. | Angle (°) | Relative intensity |
|---|---|---|---|---|---|
| 1 | 6.5 | 27.2% | 9 | 16.8 | 61.9% |
| 2 | 7.0 | 100.0% | 10 | 17.9 | 75.9% |
| 3 | 10.4 | 9.2% | 11 | 18.8 | 10.5% |
| 4 | 11.4 | 48.6% | 12 | 19.7 | 25.4% |

(continued)

| No. | Angle (°) | Relative intensity | No. | Angle (°) | Relative intensity |
|---|---|---|---|---|---|
| 5 | 13.0 | 34.8% | 13 | 22.0 | 47.2% |
| 6 | 14.1 | 81.1% | 14 | 23.4 | 66.2% |
| 7 | 15.0 | 22.8% | 15 | 24.1 | 32.1% |
| 8 ; | 16.1 | 17.8% | 16 | 26.4 | 13.9% |

(8) the X-ray powder diffraction pattern for the crystal form G, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.6 | 12.1% | 11 | 19.3 | 100.0% |
| 2 | 9.0 | 66.3% | 12 | 20.2 | 6.4% |
| 3 | 13.4 | 46.8% | 13 | 20.6 | 17.7% |
| 4 | 13.9 | 5.6% | 14 | 21.2 | 8.0% |
| 5 | 14.2 | 7.3% | 15 | 24.8 | 16.2% |
| 6 | 14.5 | 4.9% | 16 | 26.2 | 4.6% |
| 7 | 15.7 | 9.8% | 17 | 26.7 | 2.0% |
| 8 | 16.9 | 4.9% | 18 | 27.3 | 22.0% |
| 9 | 17.2 | 5.9% | 19 | 28.2 | 10.4% |
| 10 | 18.1 | 27.2% | 20 | 30.1 | 6.9% |

;

(9) the X-ray powder diffraction pattern for the crystal form J, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.4 | 50.3% | 17 | 21.3 | 36.8% |
| 2 | 9.6 | 18.9% | 18 | 22.4 | 24.3% |
| 3 | 12.9 | 30.5% | 19 | 22.8 | 55.0% |
| 4 | 14.5 | 9.1% | 20 | 23.3 | 32.5% |
| 5 | 14.8 | 13.7% | 21 | 23.9 | 30.4% |
| 6 | 15.5 | 7.8% | 22 | 24.1 | 32.5% |
| 7 | 16.5 | 11.6% | 23 | 25.2 | 42.1% |
| 8 | 16.9 | 12.0% | 24 | 25.9 | 45.9% |
| 9. | 17.7 | 26.4% | 25 | 27.5 | 8.8% |
| 10 | 18.2 | 20.5% | 26 | 28.8 | 28.2% |
| 11 | 18.7 | 100.0% | 27 | 29.6 | 12.1% |
| 12 | 19.0 | 94.6% | 28 | 30.2 | 10.4% |
| 13 | 19.2 | 82.5% | 29 | 31.9 | 7.3% |
| 14 | 19.9 | 11.5% | 30 | 35.1 | 6.2% |
| 15 | 20.3 | 11.0% | 31 | 36.8 | 6.3% |
| 16 | 20.9 | 57.0% | | | |

**4.** The crystal form A, crystal form B, crystal form C, crystal form D, crystal form F, crystal form G, crystal form H, crystal form I, or crystal form J according to claim 3, which satisfies one or more of the following conditions:

(1) the X-ray powder diffraction pattern for the crystal form A, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 6.9 | 63.6% | 9 | 17.3 | 43.2% |
| 2 | 9.7 | 18.3% | 10 | 18.2 | 52.4% |
| 3 | 10.2 | 16.2% | 11 | 18.3 | 47.0% |
| 4 | 11.2 | 60.1% | 12 | 20.6 | 32.7% |
| 5 | 12.2 | 29.2% | 13 | 21.6 | 29.8% |
| 6 | 13.7 | 100.0% | 14 | 24.3 | 27.6% |
| 7 | 15.1 | 23.2% | 15 | 24.8 | 19.8% |
| 8 | 16.4 | 20.8% | | | |

;

(2) the X-ray powder diffraction pattern for the crystal form C, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 9.7 | 34.5% | 9 | 16.8 | 21.6% |
| 2 | 10.8 | 25.5% | 10 | 18.7 | 100.0% |
| 3 | 12.5 | 29.3% | 11 | 19.6 | 55.4% |
| 4 | 13.1 | 35.7% | 12 | 19.8 | 57.1% |
| 5 | 14.1 | 48.6% | 13 | 20.7 | 69.5% |
| 6 | 14.7 | 12.3% | 14 | 21.7 | 27.0% |
| 7 | 15.2 | 22.6% | 15 | 22.4 | 45.7% |
| 8 | 15.5 | 33.4% | 16 | 24.3 | 13.1% |

;

(3) the X-ray powder diffraction pattern for the crystal form F, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 5.8 | 20.4% | 14 | 17.5 | 43.5% |
| 2 | 6.6 | 29.1% | 15 | 18.3 | 17.5% |
| 3 | 7.2 | 60.6% | 16 | 18.9 | 39.1% |
| 4 | 9.8 | 8.9% | 17 | 20.0 | 24.5% |
| 5 | 10.1 | 6.2% | 18 | 20.8 | 38.9% |
| 6 | 11.2 | 15.6% | 19 | 21.7 | 17.6% |
| 7 | 12.2 | 56.5% | 20 | 22.5 | 48.3% |
| 8 | 13.3 | 100.0% | 21 | 24.1 | 18.1% |
| 9 | 13.6 | 37.6% | 22 | 24.6 | 21.4% |
| 10 | 14.5 | 28.2% | 23 | 26.5 | 9.4% |

(continued)

| No. | Angle (°) | Relative intensity | No. | Angle (°) | Relative intensity |
|---|---|---|---|---|---|
| 11 | 15.7 | 74.0% | 24 | 27.0 | 10.4% |
| 12 | 16.2 | 30.9% | 25 | 28.9 | 3.4% |
| 13 | 16.6 | 13.9% | 26 | 35.5 | 3.3% |

;

(4) the X-ray powder diffraction pattern for the crystal form H, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 5.8 | 5.7% | 17 | 20.8 | 100.0% |
| 2 | 8.5 | 18.7% | 18 | 21.8 | 8.1% |
| 3 | 9.5 | 9.6% | 19 | 22.5 | 3.4% |
| 4 | 10.9 | 37.0% | 20 | 23.0 | 6.1% |
| 5 | 11.7 | 3.7% | 21 | 23.3 | 4.8% |
| 6 | 14.1 | 12.0% | 22 | 23.9 | 6.7% |
| 7 | 14.4 | 3.7% | 23 | 24.5 | 6.0% |
| 8 | 14.9 | 2.6% | 24 | 25.4 | 7.5% |
| 9 | 15.5 | 8.2% | 25 | 25.8 | 42.5% |
| 10 | 15.9 | 9.7% | 26 | 26.5 | 11.0% |
| 11 | 16.6 | 7.9% | 27 | 27.4 | 3.6% |
| 12 | 16.9 | 85.1% | 28 | 28.0 | 3.9% |
| 13 | 17.2 | 32.5% | 29 | 28.2 | 4.8% |
| 14 | 17.6 | 15.6% | 30 | 32.4 | 3.1% |
| 15 | 18.5 | 17.0% | 31 | 33.0 | 3.3% |
| 16 ; | 19.1 | 28.2% | | | |

(5) the X-ray powder diffraction pattern for the crystal form I, using Cu-Kα radiation and represented by 2θ angles, has positions and relative intensities of diffraction peaks as shown in the following table:

| No. | Angle (°) | Relative intensity | No. | Angle (°) | Relative intensity |
|---|---|---|---|---|---|
| 1 | 7.6 | 11.0% | 12 | 21.1 | 11.3% |
| 2 | 8.0 | 41.7% | 13 | 21.5 | 10.9% |
| 3 | 11.3 | 100.0% | 14 | 22.0 | 49.1% |
| 4 | 12.1 | 81.3% | 15 | 22.8 | 17.7% |
| 5 | 15.5 | 21.0% | 16 | 23.2 | 11.2% |
| 6 | 16.0 | 31.3% | 17 | 24.2 | 15.3% |
| 7 | 16.5 | 28.7% | 18 | 25.3 | 49.8% |
| 8 | 17.0 | 13.0% | 19 | 25.6 | 21.0% |
| 9 | 17.6 | 47.4% | 20 | 26.8 | 12.0% |
| 10 | 18.6 | 18.4% | 21 | 28.5 | 12.6% |
| 11 | 20.2 | 51.3 | 22 | 31.7 | 4.9% |

;

(6) the X-ray powder diffraction pattern for the crystal form B, using Cu-Kα radiation and represented by 2θ angles, is substantially as shown in FIG. 5;
(7) the X-ray powder diffraction pattern for the crystal form D, using Cu-Kα radiation and represented by 2θ angles, is substantially as shown in FIG. 9;
(8) the X-ray powder diffraction pattern for the crystal form G, using Cu-Kα radiation and represented by 2θ angles, is substantially as shown in FIG. 13;
(9) the X-ray powder diffraction pattern for the crystal form J, using Cu-Kα radiation and represented by 2θ angles, is substantially as shown in FIG. 20.

**5.** The crystal form A, crystal form B, crystal form C, crystal form D, crystal form F, crystal form G, crystal form H, crystal form I, or crystal form J according to claim 4, which satisfies one or more of the following conditions:

(1) the X-ray powder diffraction pattern for the crystal form A, using Cu-Kα radiation and represented by 2θ angles, is substantially as shown in FIG. 2;
(2) the differential scanning calorimetry pattern for the crystal form A is substantially as shown in FIG. 3;
(3) the thermogravimetric analysis pattern for the crystal form A is substantially as shown in FIG. 4;
(4) the X-ray powder diffraction pattern for the crystal form C, using Cu-Kα radiation and represented by 2θ angles, is substantially as shown in FIG. 6;
(5) the differential scanning calorimetry pattern for the crystal form C is substantially as shown in FIG. 7;
(6) the thermogravimetric analysis pattern for the crystal form C is substantially as shown in FIG. 8;
(7) the X-ray powder diffraction pattern for the crystal form F, using Cu-Kα radiation and represented by 2θ angles, is substantially as shown in FIG. 10;
(8) the differential scanning calorimetry pattern for the crystal form F is substantially as shown in FIG. 11;
(9) the thermogravimetric analysis pattern for the crystal form F is substantially as shown in FIG. 12;
(10) the X-ray powder diffraction pattern for the crystal form H, using Cu-Kα radiation and represented by 2θ angles, is substantially as shown in FIG. 14;
(11) the differential scanning calorimetry pattern for the crystal form H is substantially as shown in FIG. 15;
(12) the thermogravimetric analysis pattern for the crystal form H is substantially as shown in FIG. 16;
(13) the X-ray powder diffraction pattern for the crystal form I, using Cu-Kα radiation and represented by 2θ angles, is substantially as shown in FIG. 17;
(14) the differential scanning calorimetry pattern for the crystal form I is substantially as shown in FIG. 18;
(15) the thermogravimetric analysis pattern for the crystal form I is substantially as shown in FIG. 19.

**6.** A hydrochloride or sulfate of a five-membered-fused six-membered compound of formula X,

X

;

the hydrochloride of the five-membered-fused six-membered compound of formula X can be as shown in formula X-1;

X-1

;

the sulfate of the five-membered-fused six-membered compound of formula X can be as shown in formula X-2;

X-2

.

7. A preparation method for the crystal form A, crystal form C, crystal form F, crystal form H, or crystal form I of the five-membered-fused six-membered compound of formula X according to any one of claims 1 to 5, wherein

the preparation method for the crystal form A comprises the step of: slurrying the five-membered-fused six-membered compound of formula X in a ketone solvent to obtain the crystal form A; the ketone solvent is, for example, acetone; the five-membered-fused six-membered compound of formula X can be in an amorphous form;
the preparation method for the crystal form C comprises the step of: slurrying the five-membered-fused six-membered compound of formula X in a nitrile solvent to obtain the crystal form C; the nitrile solvent is, for example, acetonitrile; the five-membered-fused six-membered compound of formula X can be in an amorphous form;
the preparation method for the crystal form F comprises the step of: subjecting the five-membered-fused six-membered compound of formula X and a chloroalkane to gas-solid diffusion to obtain the crystal form F; the chloroalkane is, for example, dichloromethane; the five-membered-fused six-membered compound of formula X can be in an amorphous form;
the preparation method for the crystal form H comprises the step of: adding a benzene hydrocarbon solvent to a solution of the five-membered-fused six-membered compound of formula X in an amide solvent for crystallization to obtain the crystal form H; the amide solvent is, for example, DMF; the benzene hydrocarbon solvent is, for example, toluene; the five-membered-fused six-membered compound of formula X and the amide solvent can have a mass-to-volume ratio of 80 to 150 mg/mL, preferably 100 to 125 mg/mL, for example, 100 mg/mL or 125 mg/mL; the benzene hydrocarbon solvent and the amide solvent can have a volume ratio of (5 to 15):1, preferably (8 to 12):1, for example, 10:1; the crystallization can be performed at room temperature;
the preparation method for the crystal form I comprises the step of: adding an ether solvent to a solution of the five-membered-fused six-membered compound of formula X in a sulfoxide solvent for crystallization to obtain the crystal form I; the sulfoxide solvent is, for example, DMSO; the ether solvent is, for example, MTBE; the five-membered-fused six-membered compound of formula X and the sulfoxide solvent can have a mass-to-volume ratio of 80 to 150 mg/mL, preferably 100 to 125 mg/mL, for example, 100 mg/mL or 125 mg/mL; the ether solvent and the sulfoxide solvent can have a volume ratio of (5 to 15):1, preferably (8 to 12):1, for example, 10:1; the crystallization can be performed at room temperature.

8. A pharmaceutical composition, comprising: (1) the crystal form A, crystal form B, crystal form C, crystal form D, crystal form F, crystal form G, crystal form H, crystal form I, or crystal form J of the five-membered-fused six-membered compound of formula X according to any one of claims 1 to 5, or the hydrochloride or sulfate of the five-membered-

fused six-membered compound of formula X according to claim 6; and
(2) a pharmaceutically acceptable carrier.

**9.** A use of substance Z in the manufacture of an IRAK4 degrading agent, wherein the substance Z is the crystal form A, crystal form B, crystal form C, crystal form D, crystal form F, crystal form G, crystal form H, crystal form I, or crystal form J of the five-membered-fused six-membered compound of formula X according to any one of claims 1 to 5, or the hydrochloride or sulfate of the five-membered-fused six-membered compound of formula X according to claim 6, or the pharmaceutical composition according to claim 8.

**10.** A use of substance Z in the manufacture of a medicament for the treatment and/or prevention of a Myd88- and/or IRAK4-related disease; the substance Z is the crystal form A, crystal form B, crystal form C, crystal form D, crystal form F, crystal form G, crystal form H, crystal form I, or crystal form J of the five-membered-fused six-membered compound of formula X according to any one of claims 1 to 5, or the hydrochloride or sulfate of the five-membered-fused six-membered compound of formula X according to claim 6, or the pharmaceutical composition according to claim 8;

preferably, the IRAK4-related disease comprises one or more of a chronic lung disease, an autoimmune disease, an inflammatory disease, a tumor, a cardiovascular and cerebrovascular disease, and a central nervous system disease;
the autoimmune disease can comprise psoriasis, systemic lupus erythematosus, and rheumatoid arthritis;
the inflammatory disease can comprise an inflammatory bowel disease, such as ulcerative colitis;
the tumor can be a hematological tumor or a solid tumor; the hematological tumor can comprise large B-cell lymphoma and acute and chronic lymphocytic leukemia; the solid tumor can comprise intestinal cancer and skin cancer caused by MYD88 mutations;
the cardiovascular and cerebrovascular disease can comprise stroke and atherosclerosis;
the central nervous system disease can comprise primary central nervous system lymphoma.

Intensity
2θ (°)

FIG. 1

Intensity
2,500
2,300
2,200
2,100
2,000
1,900
1,800
1,700
1,600
1,500
1,400
1,300
1,200
1,100
1,000
900
800
700
600
500
400
300
200
0
10
20
30
40
2θ(°)


FIG. 2

Flow rate (W/g)
Temperature (°C)
262.80 °C
56.130 J/g
266.29 °C


FIG. 3

Weight (%)
Temperature (°C)
0.548 %
30.00 °C
200.00 °C


FIG. 4

Intensity
600
500
400
300
200
100
0
10
20
30
40
2θ(°)

FIG. 5

Intensity
600
500
400
300
200
100
0
10
20
30
40
2θ(°)

FIG. 6

269.44 °C
261.86 °C
43.055 J/g
266.85 °C
12.336 J/g
264.42 °C
Flow rate (W/g)
1
0
-1
-2
-3
Temperature (°C)
0
50
100
150
200
250
300

FIG. 7

Weight (%)
120
105
90
75
60
Temperature (°C)
0
50
100
150
200
250
300

FIG. 8

Intensity
1,000
900
800
700
600
500
400
300
200
100
0
10
20
30
40
2θ(°)

FIG. 9

Intensity
2,000
1,900
1,800
1,700
1,600
1,500
1,400
1,300
1,200
1,100
1,000
900
800
700
600
500
400
300
200
100
0
10
20
30
40
2θ(°)

FIG. 10

0.500
-0.125
-0.750
-1.375
-2.000
0.00
53.33
106.67
160.00
213.33
266.67
320.00
Flow rate (W/g)
Temperature (°C)
249.13 °C
46.672 J/g
260.30 °C
262.89 °C
8.5055 J/g
258.63 °C
2.1457 J/g
264.91 °C
255.54 °C

FIG. 11

130.0
107.5
85.0
62.5
40.0
0
50
100
150
200
250
300
Weight (%)
Temperature (°C)

FIG. 12

Intensity
2,000
1,900
1,800
1,700
1,600
1,500
1,400
1,300
1,200
1,100
1,000
900
800
700
600
500
400
300
200
100
0
10
20
30
40
2θ(° )

FIG. 13

Intensity
2,500
2,300
2,200
2,100
2,000
1,900
1,800
1,700
1,600
1,500
1,400
1,300
1,200
1,100
1,000
900
800
700
600
500
400
300
200
0
10
20
30
40
2θ(° )

FIG. 14

288.54 °C
70.270 J/g
290.86 °C
Flow rate (W/g)
Temperature (°C)

FIG. 15

Weight (%)
Temperature (°C)

FIG. 16

Intensity
1,000
900
800
700
600
500
400
300
200
100
0
10
20
30
40
2θ(°)

FIG. 17

Flow rate (W/g)
1
0
-1
-2
-3
279.64 °C
83.968 J/g
285.45 °C
0
50
100
150
200
250
300
Temperature (°C)

FIG. 18

110.000
103.333
96.667
90.000
83.333
76.667
70.000
Weight (%)
0.865 %
30.00 °C
150.00 °C
0
50
100
150
200
250
300
Temperature (°C)

FIG. 19

1500
1400
1300
1200
1100
1000
900
800
700
600
500
400
300
200
100
0
Intensity
10
20
30
40
2 θ (° )

FIG. 20

Cycle 1 Sorption
Cycle 1 Desorption
Change in mass (%)
Target RH (%)
0.45
0.4
0.35
0.3
0.25
0.2
0.15
0.1
0.05
0
-0.05
0
10
20
30
40
50
60
70
80
90
100
0.0035
-0.0087
0.0557
0.0538
0.1165
0.1118
0.1674
0.1594
0.2084
0.2018
0.2548
0.2461
0.2974
0.2857
0.3362
0.3233
0.3762
0.3629
0.3965

FIG. 21

Mass— Target RH
Mass (mg)
Target RH (%)
Time (min)
37
36.98
36.96
36.94
36.92
36.9
36.88
36.86
36.84
36.82
36.8
100
90
80
70
60
50
40
30
20
10
0
0
50
100
150
200
250
300
350
400

FIG. 22

Cycle 1 Sorption
Cycle 1 Desorption
Change in mass (%)
Target RH (%)
3
2.5
2
1.5
1
0.5
0
-0.5
0
10
20
30
40
50
60
70
80
90
100
0.483
0.421
0.814
0.715
1.092
0.952
1.370
1.170
1.708
1.375
1.961
1.612
2.179
1.897
2.380
2.213
2.487

FIG. 23

Mass
Target RH
Mass (mg)
Target RH (%)
Time (min)
45
44.8
44.6
44.4
44.2
44
43.8
43.6
100
90
80
70
60
50
40
30
20
10
0
0
100
200
300
400
500
600
700
800

FIG. 24

**INTERNATIONAL SEARCH REPORT**

International application No.
**PCT/CN2024/138851**

**A. CLASSIFICATION OF SUBJECT MATTER**

C07D 401/14(2006.01)i; A61K 31/519(2006.01)i; A61P 37/06(2006.01)i; A61P 29/00(2006.01)i; A61P 35/00(2006.01)i; A61P 9/00(2006.01)i; A61P 25/00(2006.01)i; A61P 17/06(2006.01)i; A61P 19/02(2006.01)i; A61P 9/10(2006.01)i

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

CNTXT, WPABS, ENTXT, VEN, Registry(STN), Caplus(STN): 杭州多域, 桥, 稠和, 氮, 酮, 癌, 肿瘤, 晶型, bridge, IRAK, fused ring, ketone, crystal, cancer, tumor, inhibitor+, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| PX | CN 118076596 A (HANGZHOU DUOYU BIOTECHNOLOGY CO., LTD.) 24 May 2024 (2024-05-24)<br>description, pages 1-121 | 1-10 |
| PX | WO 2024183650 A1 (SHANGHAI QILU PHARMACEUTICAL RESEARCH AND DEVELOPMENT CENTRE LTD.) 12 September 2024 (2024-09-12)<br>description, pages 1-117 | 1-10 |
| A | CN 114502158 A (KYMERA THERAPEUTICS, INC.) 13 May 2022 (2022-05-13)<br>description, pages 2-3 and 296-369 | 1-10 |
| A | WO 2022088551 A1 (SHANGHAI LEADINGTAC PHARMACEUTICAL CO., LTD.) 05 May 2022 (2022-05-05)<br>description, page 1, paragraph 1 and pages 4-13 | 1-10 |
| A | WO 2019099926 A1 (ARVINAS, INC.) 23 May 2019 (2019-05-23)<br>description, pages 1 and 561-576 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 February 2025** | **05 March 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/138851**

**C. DOCUMENTS CONSIDERED TO BE RELEVANT**

| Category* | Citation of document, with indication, where appropriate, of the relevant passages | Relevant to claim No. |
|---|---|---|
| A | CN 116867758 A (KYMERA THERAPEUTICS, INC.) 10 October 2023 (2023-10-10) description, pages 3-10 and 223-299 | 1-10 |

Form PCT/ISA/210 (second sheet) (July 2022)

**INTERNATIONAL SEARCH REPORT**
**Information on patent family members**

International application No.
**PCT/CN2024/138851**

| Patent document cited in search report | Publication date (day/month/year) | Patent family member(s) | Publication date (day/month/year) |
|---|---|---|---|
| CN 118076596 A | 24 May 2024 | US 2024423977 A1 | 26 December 2024 |
| | | TW 202413345 A | 01 April 2024 |
| | | AU 2023291849 A1 | 07 November 2024 |
| | | WO 2023241644 A1 | 21 December 2023 |
| WO 2024183650 A1 | 12 September 2024 | TW 202438075 A | 01 October 2024 |
| | | WO 2024183650 A9 | 19 December 2024 |
| CN 114502158 A | 13 May 2022 | EP 3989966 A1 | 04 May 2022 |
| | | EP 3989966 A4 | 27 September 2023 |
| | | JP 2022538192 A | 31 August 2022 |
| | | WO 2020264499 A1 | 30 December 2020 |
| | | US 2023069104 A1 | 02 March 2023 |
| | | BR 112021026517 A2 | 10 May 2022 |
| | | CO 2021017893 A2 | 17 January 2022 |
| | | CA 3144805 A1 | 30 December 2020 |
| | | KR 20220032063 A | 15 March 2022 |
| | | IL 289267 A | 01 February 2022 |
| | | MX 2021015995 A | 11 March 2022 |
| | | AU 2020302118 A1 | 24 February 2022 |
| WO 2022088551 A1 | 05 May 2022 | US 2023192655 A1 | 22 June 2023 |
| | | EP 4238968 A1 | 06 September 2023 |
| | | EP 4238968 A4 | 23 October 2024 |
| | | JP 2023529108 A | 07 July 2023 |
| | | JP 7474527 B2 | 25 April 2024 |
| WO 2019099926 A1 | 23 May 2019 | US 2022331297 A1 | 20 October 2022 |
| | | US 12036209 B2 | 16 July 2024 |
| | | EP 3710443 A1 | 23 September 2020 |
| | | US 2019151295 A1 | 23 May 2019 |
| | | US 11065231 B2 | 20 July 2021 |
| CN 116867758 A | 10 October 2023 | IL 303987 A | 01 August 2023 |
| | | TW 202241891 A | 01 November 2022 |
| | | CA 3202360 A1 | 07 July 2022 |
| | | AU 2021413371 A1 | 13 July 2023 |
| | | AU 2021413371 A9 | 24 October 2024 |
| | | JP 2024503300 A | 25 January 2024 |
| | | CO 2023008362 A2 | 21 July 2023 |
| | | WO 2022147465 A1 | 07 July 2022 |
| | | AR 124547 A1 | 05 April 2023 |
| | | EP 4271664 A1 | 08 November 2023 |
| | | MX 2023007852 A | 07 July 2023 |
| | | US 2023101353 A1 | 30 March 2023 |
| | | US 12150995 B2 | 26 November 2024 |
| | | KR 20230143632 A | 12 October 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023117127653
- CN 202411656766


### Non-patent literature cited in the description


- *Current Opinion in Cell Biology*, 2009, vol. 21, 1-8
- *Science*, 1996, vol. 271 (5252), 1128-31
- *Molecules*, 2016, vol. 21, 1529
- *J Biol Chem.*, 28 September 2018, vol. 293 (39), 15195-15207
- *Eur J. Immunol.*, 2008, vol. 38, 614-618
- *Molecules*, 2016, vol. 21 (11), 1529
- *Cell Chemical Biology*, 17 December 2020, vol. 27, 1-10
- *Eur. J. Immunol.*, 2008, vol. 38, 614-618
- *Expert Opinion on Therapeutic Patents*, 2019, vol. 29 (4)
- *Nature Biotechnology*, 2020, vol. 38, 1221-1223
- *ACS Med Chem Lett.*, 11 July 2019, vol. 10 (7), 1081-1085